# EUROPEAN PATENT SPECIFICATION

(11) **EP 2 097 103 B1**
(45) Date of publication and mention of the grant of the patent: **03.10.2012**
(21) Application number: 07855446.6
(22) Date of filing: 30.11.2007
(51) Int. Cl.: A61K 39/145, A61K 39/39, A61P 31/16, C07K 1/00, C07K 14/11

(54) **INFLUENZA VACCINE FORMULATION**
GRIPPEIMFPSTOFF-FORMULIERUNG
FORMULATIONS DE VACCIN CONTRE L'INFLUENZA

(30) Priority: 30.11.2006 US 868008 P
(43) Date of publication of application: 09.09.2009
(73) Proprietor: Variation Biotechnologies Inc., Gatineau QC J8Y 3B5 (CA)
(72) Inventor: OGREL, Andrei, Russell Ontario K4R 1E5 (CA)
(74) Representative: Richards, William John
(86) International application number: PCT/CA2007/002164
(87) International publication number: WO 2008/064488

(56) References cited:
- WO-A1-03/066090
- WO-A1-03/066090
- WO-A1-2006/128294
- WO-A2-2007/051036
- WO-A2-2007/056266
- US-A1- 2002 183 484
- US-A1- 2004 223 976
- KATZ ET AL: "Pathogenesis of and immunity to avian influenza A H5 viruses" BIOMEDICINE AND PHARMACOTHERAPY, ELSEVIER, PARIS, FR, vol. 54, no. 4, 1 May 2000 (2000-05-01), pages 178-187, XP005888635 ISSN: 0753-3322
- KAVERIN NIKOLAI V ET AL: "Structure of antigenic sites on the haemagglutinin molecule of H5 avian influenza virus and phenotypic variation of escape mutants" JOURNAL OF GENERAL VIROLOGY, SOCIETY FOR GENERAL MICROBIOLOGY, SPENCERS WOOD, GB, vol. 83, no. Pt 10, 1 October 2002 (2002-10-01), pages 2497-2505, XP008109058 ISSN: 0022-1317
- HA Y. ET AL.: 'X-ray structure of H5 avian and H9 swine influenza virus hemagglutinins bounds to avian and human receptor analogs' PROC. NATL. ACAD. SCI. USA vol. 98, no. 20, 25 September 2001, pages 11181 - 11186, XP002520263
- KAVERIN N.V. ET AL.: 'Structure of antigenic sites on the haemagglutinin molecule of H5 avian influenza virus and phenotypic variation of escape mutants' J. GEN. VIROLOGY vol. 83, no. PART 10, October 2002, pages 2497 - 2505, XP008109058

### SEQUENCE LISTING

<110> VARIATION BIOTECHNOLOGIES INC.
<120> INFLUENZA VACCINE FORMULATION
<130> PAT 3556W-90
<140> UNKNOWN
   <141> 2007-11-30
<150> US 60/868,008
   <151> 2006-11-30
<160> 496
<170> PatentIn version 3.4
<210> 1
   <211> 21
   <212> PRT
   <213> Influenza A virus
<400> 1
   Asn His Glu Ala Ser Ser Gly Val Ser Ser Ala Ser Pro Tyr Gln Gly
   1               5                   10                  15
   Lys Ser Ser Phe Phe
               20
<210> 2
   <211> 21
   <212> PRT
   <213> Influenza A virus
<400> 2
   Asn His Glu Ala Ser Ser Gly Val Ser Ser Ala Ser Pro Tyr Asn Gly
   1               5                   10                  15
   Lys Ser Ser Phe Phe
               20
<210> 3
   <211> 21
   <212> PRT
   <213> Influenza A virus
<400> 3
   Asn His Glu Ala Ser Ser Gly Val Ser Ser Ala Ser Pro Tyr Leu Gly
   1               5                   10                  15
   Lys Ser Ser Phe Phe
                                       20
<210> 4
   <211> 21
   <212> PRT
   <213> Influenza A virus
<400> 4
   Asn His Glu Ala Ser Ser Gly Val Ser Ser Ala Ser Pro Tyr Gln Gly
   1               5                   10                  15
   Arg Ser Ser Phe Phe
               20
<210> 5
   <211> 21
   <212> PRT
   <213> Influenza A virus
<400> 5
   Asn His Glu Ala Ser Ser Gly Val Ser Ser Ala Ser Pro Tyr Asn Gly
   1               5                   10                  15
   Arg Ser Ser Phe Phe
               20
<210> 6
   <211> 21
   <212> PRT
   <213> Influenza A virus
<400> 6
   Asn His Glu Ala Ser Ser Gly Val Ser Ser Ala Ser Pro Tyr Leu Gly
   1               5                   10                  15
   Arg Ser Ser Phe Phe
               20
<210> 7
   <211> 21
   <212> PRT
   <213> Influenza A virus
<400> 7
   Asn His Glu Ala Ser Leu Gly Val Ser Ser Ala Ser Pro Tyr Gln Gly
   1               5                   10                  15
   Lys Ser Ser Phe Phe
               20
<210> 8
   <211> 21
   <212> PRT
   <213> Influenza A virus
<400> 8
   Asn His Glu Ala Ser Leu Gly Val Ser Ser Ala Ser Pro Tyr Asn Gly
   1               5                   10                  15
   Lys Ser Ser Phe Phe
               20
<210> 9
   <211> 21
   <212> PRT
   <213> Influenza A virus
<400> 9
   Asn His Glu Ala Ser Leu Gly Val Ser Ser Ala Ser Pro Tyr Leu Gly
   1               5                   10                  15
   Lys Ser Ser Phe Phe
               20
<210> 10
   <211> 21
   <212> PRT
   <213> Influenza A virus
<400> 10
   Asn His Glu Ala Ser Leu Gly Val Ser Ser Ala Ser Pro Tyr Gln Gly
   1               5                   10                  15
   Arg Ser Ser Phe Phe
               20
<210> 11
   <211> 21
   <212> PRT
   <213> Influenza A virus
<400> 11
   Asn His Glu Ala Ser Leu Gly Val Ser Ser Ala Ser Pro Tyr Asn Gly
   1               5                   10                  15
   Arg Ser Ser Phe Phe
               20
<210> 12
   <211> 21
   <212> PRT
   <213> Influenza A virus
<400> 12
   Asn His Glu Ala Ser Leu Gly Val Ser Ser Ala Ser Pro Tyr Leu Gly
   1               5                   10                  15
   Arg Ser Ser Phe Phe
               20
<210> 13
   <211> 21
   <212> PRT
   <213> Influenza A virus
<400> 13
   Asn His Asp Ala Ser Ser Gly Val Ser Ser Ala Ser Pro Tyr Gln Gly
   1               5                   10                  15
   Lys Ser Ser Phe Phe
               20
<210> 14
   <211> 21
   <212> PRT
   <213> Influenza A virus
<400> 14
   Asn His Asp Ala Ser Ser Gly Val Ser Ser Ala Ser Pro Tyr Asn Gly
   1 5 10 15
   Lys Ser Ser Phe Phe
   20
<210> 15
   <211> 21
   <212> PRT
   <213> Influenza A virus
<400> 15
   Asn His Asp Ala Ser Ser Gly Val Ser Ser Ala Ser Pro Tyr Leu Gly
   1 5 10 15
   Lys Ser Ser Phe Phe
   20
<210> 16
   <211> 21
   <212> PRT
   <213> Influenza A virus
<400> 16
   Asn His Asp Ala Ser Ser Gly Val Ser Ser Ala Ser Pro Tyr Gln Gly
   1 5 10 15
   Arg Ser Ser Phe Phe
   20
<210> 17
   <211> 21
   <212> PRT
   <213> Influenza A virus
<400> 17
   Asn His Asp Ala Ser Ser Gly Val Ser Ser Ala Ser Pro Tyr Asn Gly
   1 5 10 15
   Arg Ser Ser Phe Phe
   20
<210> 18
   <211> 21
   <212> PRT
   <213> Influenza A virus
<400> 18
   Asn His Asp Ala Ser Ser Gly Val Ser Ser Ala Ser Pro Tyr Leu Gly
   1 5 10 15
   Arg Ser Ser Phe Phe
   20
<210> 19
   <211> 21
   <212> PRT
   <213> Influenza A virus
<400> 19
   Asn His Asp Ala Ser Leu Gly Val Ser Ser Ala Ser Pro Tyr Gln Gly
   1               5                   10                  15
   Lys Ser Ser Phe Phe
               20
<210> 20
   <211> 21
   <212> PRT
   <213> Influenza A virus
<400> 20
   Asn His Asp Ala Ser Leu Gly Val Ser Ser Ala Ser Pro Tyr Asn Gly
   1               5                   10                  15
   Lys Ser Ser Phe Phe
               20
<210> 21
   <211> 21
   <212> PRT
   <213> Influenza A virus
<400> 21
   Asn His Asp Ala Ser Leu Gly Val Ser Ser Ala Ser Pro Tyr Leu Gly
   1               5                   10                  15
   Lys Ser Ser Phe Phe
               20
<210> 22
   <211> 21
   <212> PRT
   <213> Influenza A virus
<400> 22
   Asn His Asp Ala Ser Leu Gly Val Ser Ser Ala Ser Pro Tyr Gln Gly
   1               5                   10                  15
   Arg Ser Ser Phe Phe
               20
<210> 23
   <211> 21
   <212> PRT
   <213> Influenza A virus
<400> 23
   Asn His Asp Ala Ser Leu Gly Val Ser Ser Ala Ser Pro Tyr Asn Gly
   1               5                   10                  15
   Arg Ser Ser Phe Phe
               20
<210> 24
   <211> 21
   <212> PRT
   <213> Influenza A virus
<400> 24
   Asn His Asp Ala Ser Leu Gly Val Ser Ser Ala Ser Pro Tyr Leu Gly
   1               5                   10                  15
   Arg Ser Ser Phe Phe
               20
<210> 25
   <211> 17
   <212> PRT
   <213> Influenza A virus
<400> 25
   Pro Asn Asp Ala Ala Glu Gln Thr Lys Leu Tyr Gln Asn Pro Thr Thr
   1               5                   10                  15
   Tyr
<210> 26
   <211> 17
   <212> PRT
   <213> Influenza A virus
<400> 26
   Pro Asn Asp Ala Ala Glu Gln Thr Lys Leu Tyr Gln Asn Pro Asn Thr
   1               5                   10                  15
   Tyr
<210> 27
   <211> 17
   <212> PRT
   <213> Influenza A virus
<400> 27
   Pro Asn Asp Ala Ala Glu Gln Thr Lys Leu Tyr Gln Asn Ser Thr Thr
   1 5 10 15
   Tyr
<210> 28
   <211> 17
   <212> PRT
   <213> Influenza A virus
<400> 28
   Pro Asn Asp Ala Ala Glu Gln Thr Lys Leu Tyr Gln Asn Ser Asn Thr
   1 5 10 15
   Tyr
<210> 29
   <211> 17
   <212> PRT
   <213> Influenza A virus
<400> 29
   Pro Asn Asp Ala Ala Glu Gln Thr Arg Leu Tyr Gln Asn Pro Thr Thr
   1 5 10 15
   Tyr
<210> 30
   <211> 17
   <212> PRT
   <213> Influenza A virus
<400> 30
   Pro Asn Asp Ala Ala Glu Gln Thr Arg Leu Tyr Gln Asn Pro Asn Thr
   1 5 10 15
   Tyr
<210> 31
   <211> 17
   <212> PRT
   <213> Influenza A virus
<400> 31
   Pro Asn Asp Ala Ala Glu Gln Thr Arg Leu Tyr Gln Asn Ser Thr Thr
   1 5 10 15
   Tyr
<210> 32
   <211> 17
   <212> PRT
   <213> Influenza A virus
<400> 32
   Pro Asn Asp Ala Ala Glu Gln Thr Arg Leu Tyr Gln Asn Ser Asn Thr
   1 5 10 15
   Tyr
<210> 33
   <211> 17
   <212> PRT
   <213> Influenza A virus
<400> 33
   Pro Asn Asp Ala Ala Glu Gln Ile Lys Leu Tyr Gln Asn Pro Thr Thr
   1 5 10 15
   Tyr
<210> 34
   <211> 17
   <212> PRT
   <213> Influenza A virus
<400> 34
   Pro Asn Asp Ala Ala Glu Gln Ile Lys Leu Tyr Gln Asn Pro Asn Thr
   1 5 10 15
   Tyr
<210> 35
   <211> 17
   <212> PRT
   <213> Influenza A virus
<400> 35
   Pro Asn Asp Ala Ala Glu Gln Ile Lys Leu Tyr Gln Asn Ser Thr Thr
   1 5 10 15
   Tyr
<210> 36
   <211> 17
   <212> PRT
   <213> Influenza A virus
<400> 36
   Pro Asn Asp Ala Ala Glu Gln Ile Lys Leu Tyr Gln Asn Ser Asn Thr
   1 5 10 15
   Tyr
<210> 37
   <211> 17
   <212> PRT
   <213> Influenza A virus
<400> 37
   Pro Asn Asp Ala Ala Glu Gln Ile Arg Leu Tyr Gln Asn Pro Thr Thr
   1 5 10 15
   Tyr
<210> 38
   <211> 17
   <212> PRT
   <213> Influenza A virus
<400> 38
   Pro Asn Asp Ala Ala Glu Gln Ile Arg Leu Tyr Gln Asn Pro Asn Thr
   1 5 10 15
   Tyr
<210> 39
   <211> 17
   <212> PRT
   <213> Influenza A virus
<400> 39
   Pro Asn Asp Ala Ala Glu Gln Ile Arg Leu Tyr Gln Asn Ser Thr Thr
   1 5 10 15
   Tyr
<210> 40
   <211> 17
   <212> PRT
   <213> Influenza A virus
<400> 40
   Pro Asn Asp Ala Ala Glu Gln Ile Arg Leu Tyr Gln Asn Ser Asn Thr
   1 5 10 15
   Tyr
<210> 41
   <211> 18
   <212> PRT
   <213> Influenza A virus
<400> 41
   Ser Trp Ser Asn His Glu Ala Ser Leu Ile Lys Lys Asn Ser Ala Tyr
   1               5                   10                  15
   Pro Thr
<210> 42
   <211> 18
   <212> PRT
   <213> Influenza A virus
<400> 42
   Ser Trp Ser Asn His Glu Ala Ser Leu Ile Lys Lys Asn Ser Thr Tyr
   1               5                   10                  15
   Pro Thr
<210> 43
   <211> 18
   <212> PRT
   <213> Influenza A virus
<400> 43
   Ser Trp Ser Asn His Glu Ala Ser Leu Ile Lys Lys Asn Ser Val Tyr
   1               5                   10                  15
   Pro Thr
<210> 44
   <211> 18
   <212> PRT
   <213> Influenza A virus
<400> 44
   Ser Trp Ser Asn His Glu Ala Ser Leu Ile Lys Lys Asn Asn Ala Tyr
   1               5                   10                  15
   Pro Thr
<210> 45
   <211> 18
   <212> PRT
   <213> Influenza A virus
<400> 45
   Ser Trp Ser Asn His Glu Ala Ser Leu Ile Lys Lys Asn Asn Thr Tyr
   1 5 10 15
   Pro Thr
<210> 46
   <211> 18
   <212> PRT
   <213> Influenza A virus
<400> 46
   Ser Trp Ser Asn His Glu Ala Ser Leu Ile Lys Lys Asn Asn Val Tyr
   1 5 10 15
   Pro Thr
<210> 47
   <211> 18
   <212> PRT
   <213> Influenza A virus
<400> 47
   Ser Trp Ser Asn His Asp Ala Ser Leu Ile Lys Lys Asn Ser Ala Tyr
   1 5 10 15
   Pro Thr
<210> 48
   <211> 18
   <212> PRT
   <213> Influenza A virus
<400> 48
   Ser Trp Ser Asn His Asp Ala Ser Leu Ile Lys Lys Asn Ser Thr Tyr
   1 5 10 15
   Pro Thr
<210> 49
   <211> 18
   <212> PRT
   <213> Influenza A virus
<400> 49
   Ser Trp Ser Asn His Asp Ala Ser Leu Ile Lys Lys Asn Ser Val Tyr
   1 5 10 15
   Pro Thr
<210> 50
   <211> 18
   <212> PRT
   <213> Influenza A virus
<400> 50
   Ser Trp Ser Asn His Asp Ala Ser Leu Ile Lys Lys Asn Asn Ala Tyr
   1               5                   10                  15
   Pro Thr
<210> 51
   <211> 18
   <212> PRT
   <213> Influenza A virus
<400> 51
   Ser Trp Ser Asn His Asp Ala Ser Leu Ile Lys Lys Asn Asn Thr Tyr
   1               5                   10                  15
   Pro Thr
<210> 52
   <211> 18
   <212> PRT
   <213> Influenza A virus
<400> 52
   Ser Trp Ser Asn His Asp Ala Ser Leu Ile Lys Lys Asn Asn Val Tyr
   1               5                   10                  15
   Pro Thr
<210> 53
   <211> 18
   <212> PRT
   <213> Influenza A virus
<400> 53
   Ser Trp Ser Ser His Glu Ala Ser Leu Ile Lys Lys Asn Ser Ala Tyr
   1               5                   10                  15
   Pro Thr
<210> 54
   <211> 18
   <212> PRT
   <213> Influenza A virus
<400> 54
   Ser Trp Ser Ser His Glu Ala Ser Leu Ile Lys Lys Asn Ser Thr Tyr
   1               5                   10                  15
   Pro Thr
<210> 55
   <211> 18
   <212> PRT
   <213> Influenza A virus
<400> 55
   Ser Trp Ser Ser His Glu Ala Ser Leu Ile Lys Lys Asn Ser Val Tyr
   1               5                   10                  15
   Pro Thr
<210> 56
   <211> 18
   <212> PRT
   <213> Influenza A virus
<400> 56
   Ser Trp Ser Ser His Glu Ala Ser Leu Ile Lys Lys Asn Asn Ala Tyr
   1               5                   10                  15
   Pro Thr
<210> 57
   <211> 18
   <212> PRT
   <213> Influenza A virus
<400> 57
   Ser Trp Ser Ser His Glu Ala Ser Leu Ile Lys Lys Asn Asn Thr Tyr
   1               5                   10                  15
   Pro Thr
<210> 58
   <211> 18
   <212> PRT
   <213> Influenza A virus
<400> 58
   Ser Trp Ser Ser His Glu Ala Ser Leu Ile Lys Lys Asn Asn Val Tyr
   1               5                   10                  15
   Pro Thr
<210> 59
   <211> 18
   <212> PRT
   <213> Influenza A virus
<400> 59
   Ser Trp Ser Ser His Asp Ala Ser Leu Ile Lys Lys Asn Ser Ala Tyr
   1               5                   10                  15
   Pro Thr
<210> 60
   <211> 18
   <212> PRT
   <213> Influenza A virus
<400> 60
   Ser Trp Ser Ser His Asp Ala Ser Leu Ile Lys Lys Asn Ser Thr Tyr
   1               5                   10                  15
   Pro Thr
<210> 61
   <211> 18
   <212> PRT
   <213> Influenza A virus
<400> 61
   Ser Trp Ser Ser His Asp Ala Ser Leu Ile Lys Lys Asn Ser Val Tyr
   1               5                   10                  15
   Pro Thr
<210> 62
   <211> 18
   <212> PRT
   <213> Influenza A virus
<400> 62
   Ser Trp Ser Ser His Asp Ala Ser Leu Ile Lys Lys Asn Asn Ala Tyr
   1               5                   10                  15
   Pro Thr
<210> 63
   <211> 18
   <212> PRT
   <213> Influenza A virus
<400> 63
   Ser Trp Ser Ser His Asp Ala Ser Leu Ile Lys Lys Asn Asn Thr Tyr
   1               5                   10                  15
   Pro Thr
<210> 64
   <211> 18
   <212> PRT
   <213> Influenza A virus
<400> 64
   Ser Trp Ser Ser His Asp Ala Ser Leu Ile Lys Lys Asn Asn Val Tyr
   1 5 10 15
   Pro Thr
<210> 65
   <211> 23
   <212> PRT
   <213> Influenza A virus
<400> 65
   Gly Lys Leu Ser Asp Leu Asp Gly Val Lys Pro Leu Ile Leu Leu Glu
   1 5 10 15
   Tyr Gly Asn Ser Asn Thr Lys
   20
<210> 66
   <211> 23
   <212> PRT
   <213> Influenza A virus
<400> 66
   Gly Lys Leu Ser Asp Leu Lys Gly Val Lys Pro Leu Ile Leu Leu Glu
   1 5 10 15
   Tyr Gly Asn Ser Asn Thr Lys
   20
<210> 67
   <211> 23
   <212> PRT
   <213> Influenza A virus
<400> 67
   Gly Lys Leu Ser Asp Leu Asn Gly Val Lys Pro Leu Ile Leu Leu Glu
   1 5 10 15
   Tyr Gly Asn Ser Asn Thr Lys
   20
<210> 68
   <211> 23
   <212> PRT
   <213> Influenza A virus
<400> 68
   Gly Lys Leu Ser Asp Leu Asp Gly Val Lys Pro Leu Ile Leu Leu Glu
   1 5 10 15
   Tyr Gly Asn Ser Asn Ala Lys
   20
<210> 69
   <211> 23
   <212> PRT
   <213> Influenza A virus
<400> 69
   Gly Lys Leu Ser Asp Leu Lys Gly Val Lys Pro Leu Ile Leu Leu Glu
   1 5 10 15
   Tyr Gly Asn Ser Asn Ala Lys
   20
<210> 70
   <211> 23
   <212> PRT
   <213> Influenza A virus
<400> 70
   Gly Lys Leu Ser Asp Leu Asn Gly Val Lys Pro Leu Ile Leu Leu Glu
   1 5 10 15
   Tyr Gly Asn Ser Asn Ala Lys
   20
<210> 71
   <211> 23
   <212> PRT
   <213> Influenza A virus
<400> 71
   Gly Lys Leu Ser Asp Leu Asp Gly Val Lys Pro Leu Ile Leu Leu Glu
   1 5 10 15
   Tyr Gly Asn Ser Asp Thr Lys
   20
<210> 72
   <211> 23
   <212> PRT
   <213> Influenza A virus
<400> 72
   Gly Lys Leu Ser Asp Leu Lys Gly Val Lys Pro Leu Ile Leu Leu Glu
   1 5 10 15
   Tyr Gly Asn Ser Asp Thr Lys
   20
<210> 73
   <211> 23
   <212> PRT
   <213> Influenza A virus
<400> 73
   Gly Lys Leu Ser Asp Leu Asn Gly Val Lys Pro Leu Ile Leu Leu Glu
   1               5                   10                  15
   Tyr Gly Asn Ser Asp Thr Lys
               20
<210> 74
   <211> 23
   <212> PRT
   <213> Influenza A virus
<400> 74
   Gly Lys Leu Ser Asp Leu Asp Gly Val Lys Pro Leu Ile Leu Leu Glu
   1               5                   10                  15
   Tyr Gly Asn Ser Asp Ala Lys
               20
<210> 75
   <211> 23
   <212> PRT
   <213> Influenza A virus
<400> 75
   Gly Lys Leu Ser Asp Leu Lys Gly Val Lys Pro Leu Ile Leu Leu Glu
   1               5                   10                  15
   Tyr Gly Asn Ser Asp Ala Lys
               20
<210> 76
   <211> 23
   <212> PRT
   <213> Influenza A virus
<400> 76
   Gly Lys Leu Ser Asp Leu Asn Gly Val Lys Pro Leu Ile Leu Leu Glu
   1               5                   10                  15
   Tyr Gly Asn Ser Asp Ala Lys
               20
<210> 77
   <211> 23
   <212> PRT
   <213> Influenza A virus
<400> 77
   Gly Lys Leu Ser Ser Leu Asp Gly Val Lys Pro Leu Ile Leu Leu Glu
   1               5                   10                  15
   Tyr Gly Asn Ser Asn Thr Lys
               20
<210> 78
   <211> 23
   <212> PRT
   <213> Influenza A virus
<400> 78
   Gly Lys Leu Ser Ser Leu Lys Gly Val Lys Pro Leu Ile Leu Leu Glu
   1               5                   10                  15
   Tyr Gly Asn Ser Asn Thr Lys
               20
<210> 79
   <211> 23
   <212> PRT
   <213> Influenza A virus
<400> 79
   Gly Lys Leu Ser Ser Leu Asn Gly Val Lys Pro Leu Ile Leu Leu Glu
   1               5                   10                  15
   Tyr Gly Asn Ser Asn Thr Lys
               20
<210> 80
   <211> 23
   <212> PRT
   <213> Influenza A virus
<400> 80
   Gly Lys Leu Ser Ser Leu Asp Gly Val Lys Pro Leu Ile Leu Leu Glu
   1               5                   10                  15
   Tyr Gly Asn Ser Asn Ala Lys
               20
<210> 81
   <211> 23
   <212> PRT
   <213> Influenza A virus
<400> 81
   Gly Lys Leu Ser Ser Leu Lys Gly Val Lys Pro Leu Ile Leu Leu Glu
   1               5                   10                  15
   Tyr Gly Asn Ser Asn Ala Lys
               20
<210> 82
   <211> 23
   <212> PRT
   <213> Influenza A virus
<400> 82
   Gly Lys Leu Ser Ser Leu Asn Gly Val Lys Pro Leu Ile Leu Leu Glu
   1 5 10 15
   Tyr Gly Asn Ser Asn Ala Lys
   20
<210> 83
   <211> 23
   <212> PRT
   <213> Influenza A virus
<400> 83
   Gly Lys Leu Ser Ser Leu Asp Gly Val Lys Pro Leu Ile Leu Leu Glu
   1 5 10 15
   Tyr Gly Asn Ser Asp Thr Lys
   20
<210> 84
   <211> 23
   <212> PRT
   <213> Influenza A virus

<400> 84
   Gly Lys Leu Ser Ser Leu Lys Gly Val Lys Pro Leu Ile Leu Leu Glu
   1 5 10 15
   Tyr Gly Asn Ser Asp Thr Lys
   20
<210> 85
   <211> 23
   <212> PRT
   <213> Influenza A virus
<400> 85
   Gly Lys Leu Ser Ser Leu Asn Gly Val Lys Pro Leu Ile Leu Leu Glu
   1 5 10 15
   Tyr Gly Asn Ser Asp Thr Lys
   20
<210> 86
   <211> 23
   <212> PRT
   <213> Influenza A virus
<400> 86
   Gly Lys Leu Ser Ser Leu Asp Gly Val Lys Pro Leu Ile Leu Leu Glu
   1 5 10 15
   Tyr Gly Asn Ser Asp Ala Lys
   20
<210> 87
   <211> 23
   <212> PRT
   <213> Influenza A virus
<400> 87
   Gly Lys Leu Ser Ser Leu Lys Gly Val Lys Pro Leu Ile Leu Leu Glu
   1 5 10 15
   Tyr Gly Asn Ser Asp Ala Lys
   20
<210> 88
   <211> 23
   <212> PRT
   <213> Influenza A virus
<400> 88
   Gly Lys Leu Ser Ser Leu Asn Gly Val Lys Pro Leu Ile Leu Leu Glu
   1 5 10 15
   Tyr Gly Asn Ser Asp Ala Lys
   20
<210> 89
   <211> 24
   <212> PRT
   <213> Influenza A virus
<400> 89
   Thr Ile Lys Arg Ser Tyr Asn Asn Thr Asn Gln Glu Asp Lys Pro Asn
   1 5 10 15
   Asp Ala Ile Asn Phe Glu Ser Asn
   20
<210> 90
   <211> 24
   <212> PRT
   <213> Influenza A virus
<400> 90
   Thr Ile Lys Arg Ser Tyr Asn Asn Thr Asn Gln Glu Asp Lys Pro Asn
   1 5 10 15
   Asp Ala Ile Asn Phe Glu Ser Thr
   20
<210> 91
   <211> 24
   <212> PRT
   <213> Influenza A virus
<400> 91
   Thr Ile Lys Arg Ser Tyr Asn Asn Thr Asn Gln Glu Asp Lys Pro Asn
   1               5                   10                  15
   Asp Ser Ile Asn Phe Glu Ser Asn
               20
<210> 92
   <211> 24
   <212> PRT
   <213> Influenza A virus
<400> 92
   Thr Ile Lys Arg Ser Tyr Asn Asn Thr Asn Gln Glu Asp Lys Pro Asn
   1               5                   10                  15
   Asp Ser Ile Asn Phe Glu Ser Thr
   20
<210> 93
   <211> 24
   <212> PRT
   <213> Influenza A virus
<400> 93
   Thr Ile Lys Arg Ser Tyr Asn Asn Thr Asn Gln Glu Asp Arg Pro Asn
   1               5                   10                  15
   Asp Ala Ile Asn Phe Glu Ser Asn
               20
<210> 94
   <211> 24
   <212> PRT
   <213> Influenza A virus
<400> 94
   Thr Ile Lys Arg Ser Tyr Asn Asn Thr Asn Gln Glu Asp Arg Pro Asn
   1               5                   10                  15
   Asp Ala Ile Asn Phe Glu Ser Thr
               20
<210> 95
   <211> 24
   <212> PRT
   <213> Influenza A virus
<400> 95
   Thr Ile Lys Arg Ser Tyr Asn Asn Thr Asn Gln Glu Asp Arg Pro Asn
   1               5                   10                  15
   Asp Ser Ile Asn Phe Glu Ser Asn
               20
<210> 96
   <211> 24
   <212> PRT
   <213> Influenza A virus
<400> 96
   Thr Ile Lys Arg Ser Tyr Asn Asn Thr Asn Gln Glu Asp Arg Pro Asn
   1               5                   10                  15
   Asp Ser Ile Asn Phe Glu Ser Thr
               20
<210> 97
   <211> 24
   <212> PRT
   <213> Influenza A virus
<400> 97
   Thr Ile Lys Arg Ser Tyr Asn Asn Thr Asn Val Glu Asp Lys Pro Asn
   1               5                   10                  15
   Asp Ala Ile Asn Phe Glu Ser Asn
               20
<210> 98
   <211> 24
   <212> PRT
   <213> Influenza A virus
<400> 98
   Thr Ile Lys Arg Ser Tyr Asn Asn Thr Asn Val Glu Asp Lys Pro Asn
   1               5                   10                  15
   Asp Ala Ile Asn Phe Glu Ser Thr
               20
<210> 99
   <211> 24
   <212> PRT
   <213> Influenza A virus
<400> 99
   Thr Ile Lys Arg Ser Tyr Asn Asn Thr Asn Val Glu Asp Lys Pro Asn
   1               5                   10                  15
   Asp Ser Ile Asn Phe Glu Ser Asn
               20
<210> 100
   <211> 24
   <212> PRT
   <213> Influenza A virus
<400> 100
   Thr Ile Lys Arg Ser Tyr Asn Asn Thr Asn Val Glu Asp Lys Pro Asn
   1               5                   10                  15
   Asp Ser Ile Asn Phe Glu Ser Thr
               20
<210> 101
   <211> 24
   <212> PRT
   <213> Influenza A virus
<400> 101
   Thr Ile Lys Arg Ser Tyr Asn Asn Thr Asn Val Glu Asp Arg Pro Asn
   1               5                   10                  15
   Asp Ala Ile Asn Phe Glu Ser Asn
               20
<210> 102
   <211> 24
   <212> PRT
   <213> Influenza A virus
<400> 102
   Thr Ile Lys Arg Ser Tyr Asn Asn Thr Asn Val Glu Asp Arg Pro Asn
   1               5                   10                  15
   Asp Ala Ile Asn Phe Glu Ser Thr
               20
<210> 103
   <211> 24
   <212> PRT
   <213> Influenza A virus
<400> 103
   Thr Ile Lys Arg Ser Tyr Asn Asn Thr Asn Val Glu Asp Arg Pro Asn
   1               5                   10                  15
   Asp Ser Ile Asn Phe Glu Ser Asn
               20
<210> 104
   <211> 24
   <212> PRT
   <213> Influenza A virus
<400> 104
   Thr Ile Lys Arg Ser Tyr Asn Asn Thr Asn Val Glu Asp Arg Pro Asn
   1 5 10 15
   Asp Ser Ile Asn Phe Glu Ser Thr
   20
<210> 105
   <211> 24
   <212> PRT
   <213> Influenza A virus
<400> 105
   Thr Ile Lys Arg Thr Tyr Asn Asn Thr Asn Gln Glu Asp Lys Pro Asn
   1 5 10 15
   Asp Ala Ile Asn Phe Glu Ser Asn
   20
<210> 106
   <211> 24
   <212> PRT
   <213> Influenza A virus
<400> 106
   Thr Ile Lys Arg Thr Tyr Asn Asn Thr Asn Gln Glu Asp Lys Pro Asn
   1 5 10 15
   Asp Ala Ile Asn Phe Glu Ser Thr
   20
<210> 107
   <211> 24
   <212> PRT
   <213> Influenza A virus
<400> 107
   Thr Ile Lys Arg Thr Tyr Asn Asn Thr Asn Gln Glu Asp Lys Pro Asn
   1 5 10 15
   Asp Ser Ile Asn Phe Glu Ser Asn
   20
<210> 108
   <211> 24
   <212> PRT
   <213> Influenza A virus
<400> 108
   Thr Ile Lys Arg Thr Tyr Asn Asn Thr Asn Gln Glu Asp Lys Pro Asn
   1 5 10 15
   Asp Ser Ile Asn Phe Glu Ser Thr
   20
<210> 109
   <211> 24
   <212> PRT
   <213> Influenza A virus
<400> 109
   Thr Ile Lys Arg Thr Tyr Asn Asn Thr Asn Gln Glu Asp Arg Pro Asn
   1               5                   10                  15
   Asp Ala Ile Asn Phe Glu Ser Asn
               20
<210> 110
   <211> 24
   <212> PRT
   <213> Influenza A virus
<400> 110
   Thr Ile Lys Arg Thr Tyr Asn Asn Thr Asn Gln Glu Asp Arg Pro Asn
   1               5                   10                  15
   Asp Ala Ile Asn Phe Glu Ser Thr
               20
<210> 111
   <211> 24
   <212> PRT
   <213> Influenza A virus
<400> 111
   Thr Ile Lys Arg Thr Tyr Asn Asn Thr Asn Gln Glu Asp Arg Pro Asn
   1               5                   10                  15
   Asp Ser Ile Asn Phe Glu Ser Asn
               20
<210> 112
   <211> 24
   <212> PRT
   <213> Influenza A virus
<400> 112
   Thr Ile Lys Arg Thr Tyr Asn Asn Thr Asn Gln Glu Asp Arg Pro Asn
   1               5                   10                  15
   Asp Ser Ile Asn Phe Glu Ser Thr
               20
<210> 113
   <211> 24
   <212> PRT
   <213> Influenza A virus
<400> 113
   Thr Ile Lys Arg Thr Tyr Asn Asn Thr Asn Val Glu Asp Lys Pro Asn
   1 5 10 15
   Asp Ala Ile Asn Phe Glu Ser Asn
   20
<210> 114
   <211> 24
   <212> PRT
   <213> Influenza A virus
<400> 114
   Thr Ile Lys Arg Thr Tyr Asn Asn Thr Asn Val Glu Asp Lys Pro Asn
   1 5 10 15
   Asp Ala Ile Asn Phe Glu Ser Thr
   20
<210> 115
   <211> 24
   <212> PRT
   <213> Influenza A virus
<400> 115
   Thr Ile Lys Arg Thr Tyr Asn Asn Thr Asn Val Glu Asp Lys Pro Asn
   1 5 10 15
   Asp Ser Ile Asn Phe Glu Ser Asn
   20
<210> 116
   <211> 24
   <212> PRT
   <213> Influenza A virus
<400> 116
   Thr Ile Lys Arg Thr Tyr Asn Asn Thr Asn Val Glu Asp Lys Pro Asn
   1 5 10 15
   Asp Ser Ile Asn Phe Glu Ser Thr
   20
<210> 117
   <211> 24
   <212> PRT
   <213> Influenza A virus
<400> 117
   Thr Ile Lys Arg Thr Tyr Asn Asn Thr Asn Val Glu Asp Arg Pro Asn
   1               5                   10                  15
   Asp Ala Ile Asn Phe Glu Ser Asn
               20
<210> 118
   <211> 24
   <212> PRT
   <213> Influenza A virus
<400> 118
   Thr Ile Lys Arg Thr Tyr Asn Asn Thr Asn Val Glu Asp Arg Pro Asn
   1               5                   10                  15
   Asp Ala Ile Asn Phe Glu Ser Thr
               20
<210> 119
   <211> 24
   <212> PRT
   <213> Influenza A virus
<400> 119
   Thr Ile Lys Arg Thr Tyr Asn Asn Thr Asn Val Glu Asp Arg Pro Asn
   1               5                   10                  15
   Asp Ser Ile Asn Phe Glu Ser Asn
               20
<210> 120
   <211> 24
   <212> PRT
   <213> Influenza A virus
<400> 120
   Thr Ile Lys Arg Thr Tyr Asn Asn Thr Asn Val Glu Asp Arg Pro Asn
   1               5                   10                  15
   Asp Ser Ile Asn Phe Glu Ser Thr
               20
<210> 121
   <211> 16
   <212> PRT
   <213> Influenza A virus
<400> 121
   Ile Ser Val Gly Thr Ser Thr Leu Asn Gln Arg Leu Val Pro Lys Ile
   1               5                   10                  15
<210> 122
   <211> 16
   <212> PRT
   <213> Influenza A virus
<400> 122
   Ile Ser Val Gly Thr Ser Thr Leu Asn Gln Arg Leu Val Pro Glu Ile
   1 5 10 15
<210> 123
   <211> 16
   <212> PRT
   <213> Influenza A virus
<400> 123
   Ile Ser Val Gly Thr Ser Thr Leu Asn Gln Arg Leu Val Pro Arg Ile
   1 5 10 15
<210> 124
   <211> 16
   <212> PRT
   <213> Influenza A virus
<400> 124
   Ile Ser Val Gly Thr Ser Thr Leu Asn Gln Arg Leu Ile Pro Lys Ile
   1 5 10 15
<210> 125
   <211> 16
   <212> PRT
   <213> Influenza A virus
<400> 125
   Ile Ser Val Gly Thr Ser Thr Leu Asn Gln Arg Leu Ile Pro Glu Ile
   1 5 10 15
<210> 126
   <211> 16
   <212> PRT
   <213> Influenza A virus
<400> 126
   Ile Ser Val Gly Thr Ser Thr Leu Asn Gln Arg Leu Ile Pro Arg Ile
   1 5 10 15
<210> 127
   <211> 16
   <212> PRT
   <213> Influenza A virus
<400> 127
   Ile Ser Val Gly Thr Ser Thr Leu Asn Gln Arg Ser Val Pro Lys Ile
   1               5                   10                  15
<210> 128
   <211> 16
   <212> PRT
   <213> Influenza A virus
<400> 128
   Ile Ser Val Gly Thr Ser Thr Leu Asn Gln Arg Ser Val Pro Glu Ile
   1               5                   10                  15
<210> 129
   <211> 16
   <212> PRT
   <213> Influenza A virus
<400> 129
   Ile Ser Val Gly Thr Ser Thr Leu Asn Gln Arg Ser Val Pro Arg Ile
   1               5                   10                  15
<210> 130
   <211> 16
   <212> PRT
   <213> Influenza A virus
<400> 130
   Ile Ser Val Gly Thr Ser Thr Leu Asn Gln Arg Ser Ile Pro Lys Ile
   1               5                   10                  15
<210> 131
   <211> 16
   <212> PRT
   <213> Influenza A virus
<400> 131
   Ile Ser Val Gly Thr Ser Thr Leu Asn Gln Arg Ser Ile Pro Glu Ile
   1               5                   10                  15
<210> 132
   <211> 16
   <212> PRT
   <213> Influenza A virus
<400> 132
   Ile Ser Val Gly Thr Ser Thr Leu Asn Gln Arg Ser Ile Pro Arg Ile
   1               5                   10                  15
<210> 133
   <211> 16
   <212> PRT
   <213> Influenza A virus
<400> 133
   Val Ser Val Gly Thr Ser Thr Leu Asn Gln Arg Leu Val Pro Lys Ile
   1               5                   10                  15
<210> 134
   <211> 16
   <212> PRT
   <213> Influenza A virus
<400> 134
   Val Ser Val Gly Thr Ser Thr Leu Asn Gln Arg Leu Val Pro Glu Ile
   1               5                   10                  15
<210> 135
   <211> 16
   <212> PRT
   <213> Influenza A virus
<400> 135
   Val Ser Val Gly Thr Ser Thr Leu Asn Gln Arg Leu Val Pro Arg Ile
   1               5                   10                  15
<210> 136
   <211> 16
   <212> PRT
   <213> Influenza A virus
<400> 136
   Val Ser Val Gly Thr Ser Thr Leu Asn Gln Arg Leu Ile Pro Lys Ile
   1               5                   10                  15
<210> 137
   <211> 16
   <212> PRT
   <213> Influenza A virus
<400> 137
   Val Ser Val Gly Thr Ser Thr Leu Asn Gln Arg Leu Ile Pro Glu Ile
   1               5                   10                  15
<210> 138
   <211> 16
   <212> PRT
   <213> Influenza A virus
<400> 138
   Val Ser Val Gly Thr Ser Thr Leu Asn Gln Arg Leu Ile Pro Arg Ile
   1 5 10 15
<210> 139
   <211> 16
   <212> PRT
   <213> Influenza A virus
<400> 139
   Val Ser Val Gly Thr Ser Thr Leu Asn Gln Arg Ser Val Pro Lys Ile
   1 5 10 15
<210> 140
   <211> 16
   <212> PRT
   <213> Influenza A virus
<400> 140
   Val Ser Val Gly Thr Ser Thr Leu Asn Gln Arg Ser Val Pro Glu Ile
   1 5 10 15
<210> 141
   <211> 16
   <212> PRT
   <213> Influenza A virus
<400> 141
   Val Ser Val Gly Thr Ser Thr Leu Asn Gln Arg Ser Val Pro Arg Ile
   1 5 10 15
<210> 142
   <211> 16
   <212> PRT
   <213> Influenza A virus
<400> 142
   Val Ser Val Gly Thr Ser Thr Leu Asn Gln Arg Ser Ile Pro Lys Ile
   1 5 10 15
<210> 143
   <211> 16
   <212> PRT
   <213> Influenza A virus
<400> 143
   Val Ser Val Gly Thr Ser Thr Leu Asn Gln Arg Ser Ile Pro Glu Ile
   1 5 10 15
<210> 144
   <211> 16
   <212> PRT
   <213> Influenza A virus
<400> 144
   Val Ser Val Gly Thr Ser Thr Leu Asn Gln Arg Ser Ile Pro Arg Ile
   1 5 10 15
<210> 145
   <211> 22
   <212> PRT
   <213> Influenza A virus
<400> 145
   Lys Ala Asn Pro Ala Asn Asp Leu Gly Asn Pro Met Ser Asp Glu Phe
   1 5 10 15
   Ile Asn Val Pro Glu Trp
   20
<210> 146
   <211> 22
   <212> PRT
   <213> Influenza A virus
<400> 146
   Lys Ala Asn Pro Ala Asn Asp Leu Gly Asn Pro Met Ser Asp Glu Phe
   1 5 10 15
   Leu Asn Val Pro Glu Trp
   20
<210> 147
   <211> 22
   <212> PRT
   <213> Influenza A virus
<400> 147
   Lys Ala Asn Pro Ala Asn Gly Leu Gly Asn Pro Met Ser Asp Glu Phe
   1 5 10 15
   Ile Asn Val Pro Glu Trp
   20
<210> 148
   <211> 22
   <212> PRT
   <213> Influenza A virus
<400> 148
   Lys Ala Asn Pro Ala Asn Gly Leu Gly Asn Pro Met Ser Asp Glu Phe
   1 5 10 15
   Leu Asn Val Pro Glu Trp
   20
<210> 149
   <211> 22
   <212> PRT
   <213> Influenza A virus
<400> 149
   Lys Ala Asn Pro Val Asn Asp Leu Gly Asn Pro Met Ser Asp Glu Phe
   1 5 10 15
   Ile Asn Val Pro Glu Trp
   20
<210> 150
   <211> 22
   <212> PRT
   <213> Influenza A virus
<400> 150
   Lys Ala Asn Pro Val Asn Asp Leu Gly Asn Pro Met Ser Asp Glu Phe
   1 5 10 15
   Leu Asn Val Pro Glu Trp
   20
<210> 151
   <211> 22
   <212> PRT
   <213> Influenza A virus
<400> 151
   Lys Ala Asn Pro Val Asn Gly Leu Gly Asn Pro Met Ser Asp Glu Phe
   1 5 10 15
   Ile Asn Val Pro Glu Trp
   20
<210> 152
   <211> 22
   <212> PRT
   <213> Influenza A virus
<400> 152
   Lys Ala Asn Pro Val Asn Gly Leu Gly Asn Pro Met Ser Asp Glu Phe
   1 5 10 15
   Leu Asn Val Pro Glu Trp
   20
<210> 153
   <211> 22
   <212> PRT
   <213> Influenza A virus
<400> 153
   Lys Ala Ser Pro Ala Asn Asp Leu Gly Asn Pro Met Ser Asp Glu Phe
   1 5 10 15
   Ile Asn Val Pro Glu Trp
   20
<210> 154
   <211> 22
   <212> PRT
   <213> Influenza A virus
<400> 154
   Lys Ala Ser Pro Ala Asn Asp Leu Gly Asn Pro Met Ser Asp Glu Phe
   1 5 10 15
   Leu Asn Val Pro Glu Trp
   20
<210> 155
   <211> 22
   <212> PRT
   <213> Influenza A virus
<400> 155
   Lys Ala Ser Pro Ala Asn Gly Leu Gly Asn Pro Met Ser Asp Glu Phe
   1 5 10 15
   Ile Asn Val Pro Glu Trp
   20
<210> 156
   <211> 22
   <212> PRT
   <213> Influenza A virus
<400> 156
   Lys Ala Ser Pro Ala Asn Gly Leu Gly Asn Pro Met Ser Asp Glu Phe
   1 5 10 15
   Leu Asn Val Pro Glu Trp
   20
<210> 157
   <211> 22
   <212> PRT
   <213> Influenza A virus
<400> 157
   Lys Ala Ser Pro Val Asn Asp Leu Gly Asn Pro Met Ser Asp Glu Phe
   1               5                   10                  15
   Ile Asn Val Pro Glu Trp
               20
<210> 158
   <211> 22
   <212> PRT
   <213> Influenza A virus
<400> 158
   Lys Ala Ser Pro Val Asn Asp Leu Gly Asn Pro Met Ser Asp Glu Phe
   1               5                   10                  15
   Leu Asn Val Pro Glu Trp
               20
<210> 159
   <211> 22
   <212> PRT
   <213> Influenza A virus
<400> 159
   Lys Ala Ser Pro Val Asn Gly Leu Gly Asn Pro Met Ser Asp Glu Phe
   1               5                   10                  15
   Ile Asn Val Pro Glu Trp
               20
<210> 160
   <211> 22
   <212> PRT
   <213> Influenza A virus
<400> 160
   Lys Ala Ser Pro Val Asn Gly Leu Gly Asn Pro Met Ser Asp Glu Phe
   1               5                   10                  15
   Leu Asn Val Pro Glu Trp
               20
<210> 161
   <211> 22
   <212> PRT
   <213> Influenza A virus
<400> 161
   Lys Asp Asn Pro Ala Asn Asp Leu Gly Asn Pro Met Ser Asp Glu Phe
   1               5                   10                  15
   Ile Asn Val Pro Glu Trp
               20
<210> 162
   <211> 22
   <212> PRT
   <213> Influenza A virus
<400> 162
   Lys Asp Asn Pro Ala Asn Asp Leu Gly Asn Pro Met Ser Asp Glu Phe
   1               5                   10                  15
   Leu Asn Val Pro Glu Trp
               20
<210> 163
   <211> 22
   <212> PRT
   <213> Influenza A virus
<400> 163
   Lys Asp Asn Pro Ala Asn Gly Leu Gly Asn Pro Met Ser Asp Glu Phe
   1               5                   10                  15
   Ile Asn Val Pro Glu Trp
               20
<210> 164
   <211> 22
   <212> PRT
   <213> Influenza A virus
<400> 164
   Lys Asp Asn Pro Ala Asn Gly Leu Gly Asn Pro Met Ser Asp Glu Phe
   1               5                   10                  15
   Leu Asn Val Pro Glu Trp
               20
<210> 165
   <211> 22
   <212> PRT
   <213> Influenza A virus
<400> 165
   Lys Asp Asn Pro Val Asn Asp Leu Gly Asn Pro Met Ser Asp Glu Phe
   1               5                   10                  15
   Ile Asn Val Pro Glu Trp
               20
<210> 166
   <211> 22
   <212> PRT
   <213> Influenza A virus
<400> 166
   Lys Asp Asn Pro Val Asn Asp Leu Gly Asn Pro Met Ser Asp Glu Phe
   1 5 10 15
   Leu Asn Val Pro Glu Trp
   20
<210> 167
   <211> 22
   <212> PRT
   <213> Influenza A virus
<400> 167
   Lys Asp Asn Pro Val Asn Gly Leu Gly Asn Pro Met Ser Asp Glu Phe
   1 5 10 15
   Ile Asn Val Pro Glu Trp
   20
<210> 168
   <211> 22
   <212> PRT
   <213> Influenza A virus
<400> 168
   Lys Asp Asn Pro Val Asn Gly Leu Gly Asn Pro Met Ser Asp Glu Phe
   1 5 10 15
   Leu Asn Val Pro Glu Trp
   20
<210> 169
   <211> 22
   <212> PRT
   <213> Influenza A virus
<400> 169
   Lys Asp Ser Pro Ala Asn Asp Leu Gly Asn Pro Met Ser Asp Glu Phe
   1 5 10 15
   Ile Asn Val Pro Glu Trp
   20
<210> 170
   <211> 22
   <212> PRT
   <213> Influenza A virus
<400> 170
   Lys Asp Ser Pro Ala Asn Asp Leu Gly Asn Pro Met Ser Asp Glu Phe
   1               5                   10                  15
   Leu Asn Val Pro Glu Trp
               20
<210> 171
   <211> 22
   <212> PRT
   <213> Influenza A virus
<400> 171
   Lys Asp Ser Pro Ala Asn Gly Leu Gly Asn Pro Met Ser Asp Glu Phe
   1               5                   10                  15
   Ile Asn Val Pro Glu Trp
               20
<210> 172
   <211> 22
   <212> PRT
   <213> Influenza A virus
<400> 172
   Lys Asp Ser Pro Ala Asn Gly Leu Gly Asn Pro Met Ser Asp Glu Phe
   1               5                   10                  15
   Leu Asn Val Pro Glu Trp
                                         20

<210> 173
   <211> 22
   <212> PRT
   <213> Influenza A virus
<400> 173
   Lys Asp Ser Pro Val Asn Asp Leu Gly Asn Pro Met Ser Asp Glu Phe
   1               5                   10                  15
   Ile Asn Val Pro Glu Trp
               20
<210> 174
   <211> 22
   <212> PRT
   <213> Influenza A virus
<400> 174
   Lys Asp Ser Pro Val Asn Asp Leu Gly Asn Pro Met Ser Asp Glu Phe
   1 5 10 15
   Leu Asn Val Pro Glu Trp
   20
<210> 175
   <211> 22
   <212> PRT
   <213> Influenza A virus
<400> 175
   Lys Asp Ser Pro Val Asn Gly Leu Gly Asn Pro Met Ser Asp Glu Phe
   1 5 10 15
   Ile Asn Val Pro Glu Trp
   20
<210> 176
   <211> 22
   <212> PRT
   <213> Influenza A virus
<400> 176
   Lys Asp Ser Pro Val Asn Gly Leu Gly Asn Pro Met Ser Asp Glu Phe
   1 5 10 15
   Leu Asn Val Pro Glu Trp
   20
<210> 177
   <211> 22
   <212> PRT
   <213> Influenza A virus
<400> 177
   Pro Tyr Gln Gly Lys Ser Ser Phe Phe Arg Asn Val Val Trp Leu Ile
   1 5 10 15
   Lys Lys Asn Ser Ala Tyr
   20
<210> 178
   <211> 22
   <212> PRT
   <213> Influenza A virus
<400> 178
   Pro Tyr Gln Gly Lys Ser Ser Phe Phe Arg Asn Val Val Trp Leu Ile
   1 5 10 15
   Lys Lys Asn Ser Thr Tyr
   20
<210> 179
   <211> 22
   <212> PRT
   <213> Influenza A virus
<400> 179
   Pro Tyr Gln Gly Lys Ser Ser Phe Phe Arg Asn Val Val Trp Leu Ile
   1 5 10 15
   Lys Lys Asn Ser Val Tyr
   20
<210> 180
   <211> 22
   <212> PRT
   <213> Influenza A virus
<400> 180
   Pro Tyr Asn Gly Lys Ser Ser Phe Phe Arg Asn Val Val Trp Leu Ile
   1 5 10 15
   Lys Lys Asn Ser Ala Tyr
   20
<210> 181
   <211> 22
   <212> PRT
   <213> Influenza A virus
<400> 181
   Pro Tyr Asn Gly Lys Ser Ser Phe Phe Arg Asn Val Val Trp Leu Ile
   1 5 10 15
   Lys Lys Asn Ser Thr Tyr
   20
<210> 182
   <211> 22
   <212> PRT
   <213> Influenza A virus
<400> 182
   Pro Tyr Asn Gly Lys Ser Ser Phe Phe Arg Asn Val Val Trp Leu Ile
   1 5 10 15
   Lys Lys Asn Ser Val Tyr
   20
<210> 183
   <211> 22
   <212> PRT
   <213> Influenza A virus
<400> 183
   Pro Tyr Leu Gly Lys Ser Ser Phe Phe Arg Asn Val Val Trp Leu Ile
   1 5 10 15
   Lys Lys Asn Ser Ala Tyr
   20
<210> 184
   <211> 22
   <212> PRT
   <213> Influenza A virus
<400> 184
   Pro Tyr Leu Gly Lys Ser Ser Phe Phe Arg Asn Val Val Trp Leu Ile
   1 5 10 15
   Lys Lys Asn Ser Thr Tyr
   20
<210> 185
   <211> 22
   <212> PRT
   <213> Influenza A virus
<400> 185
   Pro Tyr Leu Gly Lys Ser Ser Phe Phe Arg Asn Val Val Trp Leu Ile
   1 5 10 15
   Lys Lys Asn Ser Val Tyr
   20
<210> 186
   <211> 22
   <212> PRT
   <213> Influenza A virus
<400> 186
   Pro Tyr Gln Gly Lys Ser Ser Phe Phe Arg Asn Val Val Trp Leu Ile
   1 5 10 15
   Lys Lys Asn Asn Ala Tyr
   20
<210> 187
   <211> 22
   <212> PRT
   <213> Influenza A virus
<400> 187
   Pro Tyr Gln Gly Lys Ser Ser Phe Phe Arg Asn Val Val Trp Leu Ile
   1 5 10 15
   Lys Lys Asn Asn Thr Tyr
   20
<210> 188
   <211> 22
   <212> PRT
   <213> Influenza A virus
<400> 188
   Pro Tyr Gln Gly Lys Ser Ser Phe Phe Arg Asn Val Val Trp Leu Ile
   1               5                   10                  15
   Lys Lys Asn Asn Val Tyr
               20
<210> 189
   <211> 22
   <212> PRT
   <213> Influenza A virus
<400> 189
   Pro Tyr Asn Gly Lys Ser Ser Phe Phe Arg Asn Val Val Trp Leu Ile
   1               5                   10                  15
   Lys Lys Asn Asn Ala Tyr
               20
<210> 190
   <211> 22
   <212> PRT
   <213> Influenza A virus
<400> 190
   Pro Tyr Asn Gly Lys Ser Ser Phe Phe Arg Asn Val Val Trp Leu Ile
   1               5                   10                  15
   Lys Lys Asn Asn Thr Tyr
               20
<210> 191
   <211> 22
   <212> PRT
   <213> Influenza A virus
<400> 191
   Pro Tyr Asn Gly Lys Ser Ser Phe Phe Arg Asn Val Val Trp Leu Ile
   1               5                   10                  15
   Lys Lys Asn Asn Val Tyr
               20
<210> 192
   <211> 22
   <212> PRT
   <213> Influenza A virus
<400> 192
   Pro Tyr Leu Gly Lys Ser Ser Phe Phe Arg Asn Val Val Trp Leu Ile
   1               5                   10                  15
   Lys Lys Asn Asn Ala Tyr
               20
<210> 193
   <211> 22
   <212> PRT
   <213> Influenza A virus
<400> 193
   Pro Tyr Leu Gly Lys Ser Ser Phe Phe Arg Asn Val Val Trp Leu Ile
   1               5                   10                  15
   Lys Lys Asn Asn Thr Tyr
               20
<210> 194
   <211> 22
   <212> PRT
   <213> Influenza A virus
<400> 194
   Pro Tyr Leu Gly Lys Ser Ser Phe Phe Arg Asn Val Val Trp Leu Ile
   1               5                   10                  15
   Lys Lys Asn Asn Val Tyr
               20
<210> 195
   <211> 22
   <212> PRT
   <213> Influenza A virus
<400> 195
   Pro Tyr Gln Gly Arg Ser Ser Phe Phe Arg Asn Val Val Trp Leu Ile
   1               5                   10                  15
   Lys Lys Asn Ser Ala Tyr
               20
<210> 196
   <211> 22
   <212> PRT
   <213> Influenza A virus
<400> 196
   Pro Tyr Gln Gly Arg Ser Ser Phe Phe Arg Asn Val Val Trp Leu Ile
   1               5                   10                  15
   Lys Lys Asn Ser Thr Tyr
               20
<210> 197
   <211> 22
   <212> PRT
   <213> Influenza A virus
<400> 197
   Pro Tyr Gln Gly Arg Ser Ser Phe Phe Arg Asn Val Val Trp Leu Ile
   1               5                   10                  15
   Lys Lys Asn Ser Val Tyr
               20
<210> 198
   <211> 22
   <212> PRT
   <213> Influenza A virus
<400> 198
   Pro Tyr Asn Gly Arg Ser Ser Phe Phe Arg Asn Val Val Trp Leu Ile
   1               5                   10                  15
   Lys Lys Asn Ser Ala Tyr
               20
<210> 199
   <211> 22
   <212> PRT
   <213> Influenza A virus
<400> 199
   Pro Tyr Asn Gly Arg Ser Ser Phe Phe Arg Asn Val Val Trp Leu Ile
   1               5                   10                  15
   Lys Lys Asn Ser Thr Tyr
               20
<210> 200
   <211> 22
   <212> PRT
   <213> Influenza A virus
<400> 200
   Pro Tyr Asn Gly Arg Ser Ser Phe Phe Arg Asn Val Val Trp Leu Ile
   1               5                   10                  15
   Lys Lys Asn Ser Val Tyr
               20
<210> 201
   <211> 22
   <212> PRT
   <213> Influenza A virus
<400> 201
   Pro Tyr Leu Gly Arg Ser Ser Phe Phe Arg Asn Val Val Trp Leu Ile
   1               5                   10                  15
   Lys Lys Asn Ser Ala Tyr
               20
<210> 202
   <211> 22
   <212> PRT
   <213> Influenza A virus
<400> 202
   Pro Tyr Leu Gly Arg Ser Ser Phe Phe Arg Asn Val Val Trp Leu Ile
   1               5                   10                  15
   Lys Lys Asn Ser Thr Tyr
               20
<210> 203
   <211> 22
   <212> PRT
   <213> Influenza A virus
<400> 203
   Pro Tyr Leu Gly Arg Ser Ser Phe Phe Arg Asn Val Val Trp Leu Ile
   1               5                   10                  15
   Lys Lys Asn Ser Val Tyr
               20
<210> 204
   <211> 22
   <212> PRT
   <213> Influenza A virus
<400> 204
   Pro Tyr Gln Gly Arg Ser Ser Phe Phe Arg Asn Val Val Trp Leu Ile
   1               5                   10                  15
   Lys Lys Asn Asn Ala Tyr
               20
<210> 205
   <211> 22
   <212> PRT
   <213> Influenza A virus
<400> 205
   Pro Tyr Gln Gly Arg Ser Ser Phe Phe Arg Asn Val Val Trp Leu Ile
   1 5 10 15
   Lys Lys Asn Asn Thr Tyr
   20
<210> 206
   <211> 22
   <212> PRT
   <213> Influenza A virus
<400> 206
   Pro Tyr Gln Gly Arg Ser Ser Phe Phe Arg Asn Val Val Trp Leu Ile
   1 5 10 15
   Lys Lys Asn Asn Val Tyr
   20
<210> 207
   <211> 22
   <212> PRT
   <213> Influenza A virus
<400> 207
   Pro Tyr Asn Gly Arg Ser Ser Phe Phe Arg Asn Val Val Trp Leu Ile
   1 5 10 15
   Lys Lys Asn Asn Ala Tyr
   20
<210> 208
   <211> 22
   <212> PRT
   <213> Influenza A virus
<400> 208
   Pro Tyr Asn Gly Arg Ser Ser Phe Phe Arg Asn Val Val Trp Leu Ile
   1 5 10 15
   Lys Lys Asn Asn Thr Tyr
   20
<210> 209
   <211> 22
   <212> PRT
   <213> Influenza A virus
<400> 209
   Pro Tyr Asn Gly Arg Ser Ser Phe Phe Arg Asn Val Val Trp Leu Ile
   1 5 10 15
   Lys Lys Asn Asn Val Tyr
   20
<210> 210
   <211> 22
   <212> PRT
   <213> Influenza A virus
<400> 210
   Pro Tyr Leu Gly Arg Ser Ser Phe Phe Arg Asn Val Val Trp Leu Ile
   1 5 10 15
   Lys Lys Asn Asn Ala Tyr
   20
<210> 211
   <211> 22
   <212> PRT
   <213> Influenza A virus
<400> 211
   Pro Tyr Leu Gly Arg Ser Ser Phe Phe Arg Asn Val Val Trp Leu Ile
   1 5 10 15
   Lys Lys Asn Asn Thr Tyr
   20
<210> 212
   <211> 22
   <212> PRT
   <213> Influenza A virus
<400> 212
   Pro Tyr Leu Gly Arg Ser Ser Phe Phe Arg Asn Val Val Trp Leu Ile
   1 5 10 15
   Lys Lys Asn Asn Val Tyr
   20
<210> 213
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 213
   Pro Tyr Gln Gly Lys Ser Ser Phe Phe Arg Asn Val Val Trp Leu Ile
   1 5 10 15
   Lys Lys Asn Ser Ala Tyr
   20
<210> 214
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 214
   Pro Tyr Gln Gly Lys Ser Ser Phe Phe Arg Asn Val Val Trp Leu Ile
   1               5                   10                  15
   Lys Lys Asn Ser Thr Tyr
               20
<210> 215
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 215
   Pro Tyr Gln Gly Lys Ser Ser Phe Phe Arg Asn Val Val Trp Leu Ile
   1               5                   10                  15
   Lys Lys Asn Ser Val Tyr
               20
<210> 216
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 216
   Pro Tyr Asn Gly Lys Ser Ser Phe Phe Arg Asn Val Val Trp Leu Ile
   1               5                   10                  15
   Lys Lys Asn Ser Ala Tyr
               20
<210> 217
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 217
   Pro Tyr Asn Gly Lys Ser Ser Phe Phe Arg Asn Val Val Trp Leu Ile
   1               5                   10                  15
   Lys Lys Asn Ser Thr Tyr
               20
<210> 218
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 218
   Pro Tyr Asn Gly Lys Ser Ser Phe Phe Arg Asn Val Val Trp Leu Ile
   1               5                   10                  15
   Lys Lys Asn Ser Val Tyr
               20
<210> 219
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 219
   Pro Tyr Leu Gly Lys Ser Ser Phe Phe Arg Asn Val Val Trp Leu Ile
   1               5                   10                  15
   Lys Lys Asn Ser Ala Tyr
               20
<210> 220
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 220
   Pro Tyr Leu Gly Lys Ser Ser Phe Phe Arg Asn Val Val Trp Leu Ile
   1 5 10 15
   Lys Lys Asn Ser Thr Tyr
   20
<210> 221
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 221
   Pro Tyr Leu Gly Lys Ser Ser Phe Phe Arg Asn Val Val Trp Leu Ile
   1 5 10 15
   Lys Lys Asn Ser Val Tyr
   20
<210> 222
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 222
   Pro Tyr Gln Gly Lys Ser Ser Phe Phe Arg Asn Val Val Trp Leu Ile
   1 5 10 15
   Lys Lys Asn Asn Ala Tyr
   20
<210> 223
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 223
   Pro Tyr Gln Gly Lys Ser Ser Phe Phe Arg Asn Val Val Trp Leu Ile
   1 5 10 15
   Lys Lys Asn Asn Thr Tyr
   20
<210> 224
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 224
   Pro Tyr Gln Gly Lys Ser Ser Phe Phe Arg Asn Val Val Trp Leu Ile
   1 5 10 15
   Lys Lys Asn Asn Val Tyr
   20
<210> 225
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 225
   Pro Tyr Asn Gly Lys Ser Ser Phe Phe Arg Asn Val Val Trp Leu Ile
   1 5 10 15
   Lys Lys Asn Asn Ala Tyr
   20
<210> 226
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 226
   Pro Tyr Asn Gly Lys Ser Ser Phe Phe Arg Asn Val Val Trp Leu Ile
   1 5 10 15
   Lys Lys Asn Asn Thr Tyr
   20
<210> 227
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 227
   Pro Tyr Asn Gly Lys Ser Ser Phe Phe Arg Asn Val Val Trp Leu Ile
   1 5 10 15
   Lys Lys Asn Asn Val Tyr
   20
<210> 228
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 228
   Pro Tyr Leu Gly Lys Ser Ser Phe Phe Arg Asn Val Val Trp Leu Ile
   1 5 10 15
   Lys Lys Asn Asn Ala Tyr
   20
<210> 229
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 229
   Pro Tyr Leu Gly Lys Ser Ser Phe Phe Arg Asn Val Val Trp Leu Ile
   1 5 10 15
   Lys Lys Asn Asn Thr Tyr
   20
<210> 230
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 230
   Pro Tyr Leu Gly Lys Ser Ser Phe Phe Arg Asn Val Val Trp Leu Ile
   1               5                   10                  15
   Lys Lys Asn Asn Val Tyr
               20
<210> 231
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 231
   Pro Tyr Gln Gly Arg Ser Ser Phe Phe Arg Asn Val Val Trp Leu Ile
   1               5                   10                  15
   Lys Lys Asn Ser Ala Tyr
               20
<210> 232
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 232
   Pro Tyr Gln Gly Arg Ser Ser Phe Phe Arg Asn Val Val Trp Leu Ile
   1               5                   10                  15
   Lys Lys Asn Ser Thr Tyr
                                              20
<210> 233
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 233
   Pro Tyr Gln Gly Arg Ser Ser Phe Phe Arg Asn Val Val Trp Leu Ile
   1               5                   10                  15
   Lys Lys Asn Ser Val Tyr
               20
<210> 234
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 234
   Pro Tyr Asn Gly Arg Ser Ser Phe Phe Arg Asn Val Val Trp Leu Ile
   1               5                   10                  15
   Lys Lys Asn Ser Ala Tyr
               20
<210> 235
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 235
   Pro Tyr Asn Gly Arg Ser Ser Phe Phe Arg Asn Val Val Trp Leu Ile
   1               5                   10                  15
   Lys Lys Asn Ser Thr Tyr
                                   20
<210> 236
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 236
   Pro Tyr Asn Gly Arg Ser Ser Phe Phe Arg Asn Val Val Trp Leu Ile
   1               5                   10                  15
   Lys Lys Asn Ser Val Tyr
               20
<210> 237
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 237
   Pro Tyr Leu Gly Arg Ser Ser Phe Phe Arg Asn Val Val Trp Leu Ile
   1               5                   10                  15
   Lys Lys Asn Ser Ala Tyr
               20
<210> 238
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 238
   Pro Tyr Leu Gly Arg Ser Ser Phe Phe Arg Asn Val Val Trp Leu Ile
   1               5                   10                  15
   Lys Lys Asn Ser Thr Tyr
               20
<210> 239
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 239
   Pro Tyr Leu Gly Arg Ser Ser Phe Phe Arg Asn Val Val Trp Leu Ile
   1               5                   10                  15
   Lys Lys Asn Ser Val Tyr
               20
<210> 240
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 240
   Pro Tyr Gln Gly Arg Ser Ser Phe Phe Arg Asn Val Val Trp Leu Ile
   1               5                   10                  15
   Lys Lys Asn Asn Ala Tyr
               20
<210> 241
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 241
   Pro Tyr Gln Gly Arg Ser Ser Phe Phe Arg Asn Val Val Trp Leu Ile
   1               5                   10                  15
   Lys Lys Asn Asn Thr Tyr
               20
<210> 242
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 242
   Pro Tyr Gln Gly Arg Ser Ser Phe Phe Arg Asn Val Val Trp Leu Ile
   1 5 10 15
   Lys Lys Asn Asn Val Tyr
   20
<210> 243
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 243
   Pro Tyr Asn Gly Arg Ser Ser Phe Phe Arg Asn Val Val Trp Leu Ile
   1 5 10 15
   Lys Lys Asn Asn Ala Tyr
   20
<210> 244
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 244
   Pro Tyr Asn Gly Arg Ser Ser Phe Phe Arg Asn Val Val Trp Leu Ile
   1 5 10 15
   Lys Lys Asn Asn Thr Tyr
   20

<210> 245
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 245
   Pro Tyr Asn Gly Arg Ser Ser Phe Phe Arg Asn Val Val Trp Leu Ile
   1               5                   10                  15
   Lys Lys Asn Asn Val Tyr
               20
<210> 246
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 246
   Pro Tyr Leu Gly Arg Ser Ser Phe Phe Arg Asn Val Val Trp Leu Ile
   1               5                   10                  15
   Lys Lys Asn Asn Ala Tyr
               20
<210> 247
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 247
   Pro Tyr Leu Gly Arg Ser Ser Phe Phe Arg Asn Val Val Trp Leu Ile
   1               5                   10                  15
   Lys Lys Asn Asn Thr Tyr
               20
<210> 248
   <211> 22
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 248
   Pro Tyr Leu Gly Arg Ser Ser Phe Phe Arg Asn Val Val Trp Leu Ile
   1 5 10 15
   Lys Lys Asn Asn Val Tyr
   20
<210> 249
   <211> 22
   <212> PRT
   <213> Influenza A virus
<400> 249
   Asn His Glu Ala Ser Ser Gly Val Ser Ser Ala Ser Pro Tyr Gln Gly
   1 5 10 15
   Lys Ser Ser Phe Phe Gly
   20
<210> 250
   <211> 22
   <212> PRT
   <213> Influenza A virus
<400> 250
   Asn His Glu Ala Ser Ser Gly Val Ser Ser Ala Ser Pro Tyr Asn Gly
   1 5 10 15
   Lys Ser Ser Phe Phe Gly
   20
<210> 251
   <211> 22
   <212> PRT
   <213> Influenza A virus
<400> 251
   Asn His Glu Ala Ser Ser Gly Val Ser Ser Ala Ser Pro Tyr Leu Gly
   1 5 10 15
   Lys Ser Ser Phe Phe Gly
   20
<210> 252
   <211> 22
   <212> PRT
   <213> Influenza A virus
<400> 252
   Asn His Glu Ala Ser Ser Gly Val Ser Ser Ala Ser Pro Tyr Gln Gly
   1 5 10 15
   Arg Ser Ser Phe Phe Gly
   20
<210> 253
   <211> 22
   <212> PRT
   <213> Influenza A virus
<400> 253
   Asn His Glu Ala Ser Ser Gly Val Ser Ser Ala Ser Pro Tyr Asn Gly
   1 5 10 15
   Arg Ser Ser Phe Phe Gly
   20
<210> 254
   <211> 22
   <212> PRT
   <213> Influenza A virus
<400> 254
   Asn His Glu Ala Ser Ser Gly Val Ser Ser Ala Ser Pro Tyr Leu Gly
   1 5 10 15
   Arg Ser Ser Phe Phe Gly
   20
<210> 255
   <211> 22
   <212> PRT
   <213> Influenza A virus
<400> 255
   Asn His Glu Ala Ser Leu Gly Val Ser Ser Ala Ser Pro Tyr Gln Gly
   1 5 10 15
   Lys Ser Ser Phe Phe Gly
   20
<210> 256
   <211> 22
   <212> PRT
   <213> Influenza A virus
<400> 256
   Asn His Glu Ala Ser Leu Gly Val Ser Ser Ala Ser Pro Tyr Asn Gly
   1 5 10 15
   Lys Ser Ser Phe Phe Gly
   20
<210> 257
   <211> 22
   <212> PRT
   <213> Influenza A virus
<400> 257
   Asn His Glu Ala Ser Leu Gly Val Ser Ser Ala Ser Pro Tyr Leu Gly
   1               5                   10                  15
   Lys Ser Ser Phe Phe Gly
               20
<210> 258
   <211> 22
   <212> PRT
   <213> Influenza A virus
<400> 258
   Asn His Glu Ala Ser Leu Gly Val Ser Ser Ala Ser Pro Tyr Gln Gly
   1               5                   10                  15
   Arg Ser Ser Phe Phe Gly
               20
<210> 259
   <211> 22
   <212> PRT
   <213> Influenza A virus
<400> 259
   Asn His Glu Ala Ser Leu Gly Val Ser Ser Ala Ser Pro Tyr Asn Gly
   1               5                   10                  15
   Arg Ser Ser Phe Phe Gly
               20
<210> 260
   <211> 22
   <212> PRT
   <213> Influenza A virus
<400> 260
   Asn His Glu Ala Ser Leu Gly Val Ser Ser Ala Ser Pro Tyr Leu Gly
   1               5                   10                  15
   Arg Ser Ser Phe Phe Gly
               20
<210> 261
   <211> 22
   <212> PRT
   <213> Influenza A virus
<400> 261
   Asn His Asp Ala Ser Ser Gly Val Ser Ser Ala Ser Pro Tyr Gln Gly
   1               5                   10                  15
   Lys Ser Ser Phe Phe Gly
               20
<210> 262
   <211> 22
   <212> PRT
   <213> Influenza A virus
<400> 262
   Asn His Asp Ala Ser Ser Gly Val Ser Ser Ala Ser Pro Tyr Asn Gly
   1               5                   10                  15
   Lys Ser Ser Phe Phe Gly
               20
<210> 263
   <211> 22
   <212> PRT
   <213> Influenza A virus
<400> 263
   Asn His Asp Ala Ser Ser Gly Val Ser Ser Ala Ser Pro Tyr Leu Gly
   1               5                   10                  15
   Lys Ser Ser Phe Phe Gly
               20
<210> 264
   <211> 22
   <212> PRT
   <213> Influenza A virus
<400> 264
   Asn His Asp Ala Ser Ser Gly Val Ser Ser Ala Ser Pro Tyr Gln Gly
   1               5                   10                  15
   Arg Ser Ser Phe Phe Gly
               20
<210> 265
   <211> 22
   <212> PRT
   <213> Influenza A virus
<400> 265
   Asn His Asp Ala Ser Ser Gly Val Ser Ser Ala Ser Pro Tyr Asn Gly
   1               5                   10                  15
   Arg Ser Ser Phe Phe Gly
               20
<210> 266
   <211> 22
   <212> PRT
   <213> Influenza A virus
<400> 266
   Asn His Asp Ala Ser Ser Gly Val Ser Ser Ala Ser Pro Tyr Leu Gly
   1 5 10 15
   Arg Ser Ser Phe Phe Gly
   20
<210> 267
   <211> 22
   <212> PRT
   <213> Influenza A virus
<400> 267
   Asn His Asp Ala Ser Leu Gly Val Ser Ser Ala Ser Pro Tyr Gln Gly
   1 5 10 15
   Lys Ser Ser Phe Phe Gly
   20
<210> 268
   <211> 22
   <212> PRT
   <213> Influenza A virus
<400> 268
   Asn His Asp Ala Ser Leu Gly Val Ser Ser Ala Ser Pro Tyr Asn Gly
   1 5 10 15
   Lys Ser Ser Phe Phe Gly
   20
<210> 269
   <211> 22
   <212> PRT
   <213> Influenza A virus
<400> 269
   Asn His Asp Ala Ser Leu Gly Val Ser Ser Ala Ser Pro Tyr Leu Gly
   1 5 10 15
   Lys Ser Ser Phe Phe Gly
   20
<210> 270
   <211> 22
   <212> PRT
   <213> Influenza A virus
<400> 270
   Asn His Asp Ala Ser Leu Gly Val Ser Ser Ala Ser Pro Tyr Gln Gly
   1 5 10 15
   Arg Ser Ser Phe Phe Gly
   20
<210> 271
   <211> 22
   <212> PRT
   <213> Influenza A virus
<400> 271
   Asn His Asp Ala Ser Leu Gly Val Ser Ser Ala Ser Pro Tyr Asn Gly
   1 5 10 15
   Arg Ser Ser Phe Phe Gly
   20
<210> 272
   <211> 22
   <212> PRT
   <213> Influenza A virus
<400> 272
   Asn His Asp Ala Ser Leu Gly Val Ser Ser Ala Ser Pro Tyr Leu Gly
   1 5 10 15
   Arg Ser Ser Phe Phe Gly
   20
<210> 273
   <211> 18
   <212> PRT
   <213> Influenza A virus
<400> 273
   Pro Asn Asp Ala Ala Glu Gln Thr Lys Leu Tyr Gln Asn Pro Thr Thr
   1 5 10 15
   Tyr Gly
<210> 274
   <211> 18
   <212> PRT
   <213> Influenza A virus
<400> 274
   Pro Asn Asp Ala Ala Glu Gln Thr Lys Leu Tyr Gln Asn Pro Asn Thr
   1 5 10 15
   Tyr Gly
<210> 275
   <211> 18
   <212> PRT
   <213> Influenza A virus
<400> 275
   Pro Asn Asp Ala Ala Glu Gln Thr Lys Leu Tyr Gln Asn Ser Thr Thr
   1               5                   10                  15
   Tyr Gly
<210> 276
   <211> 18
   <212> PRT
   <213> Influenza A virus
<400> 276
   Pro Asn Asp Ala Ala Glu Gln Thr Lys Leu Tyr Gln Asn Ser Asn Thr
   1               5                   10                  15
   Tyr Gly
<210> 277
   <211> 18
   <212> PRT
   <213> Influenza A virus
<400> 277
   Pro Asn Asp Ala Ala Glu Gln Thr Arg Leu Tyr Gln Asn Pro Thr Thr
   1               5                   10                  15
   Tyr Gly
<210> 278
   <211> 18
   <212> PRT
   <213> Influenza A virus
<400> 278
   Pro Asn Asp Ala Ala Glu Gln Thr Arg Leu Tyr Gln Asn Pro Asn Thr
   1               5                   10                  15
   Tyr Gly
<210> 279
   <211> 18
   <212> PRT
   <213> Influenza A virus
<400> 279
   Pro Asn Asp Ala Ala Glu Gln Thr Arg Leu Tyr Gln Asn Ser Thr Thr
   1 5 10 15
   Tyr Gly
<210> 280
   <211> 18
   <212> PRT
   <213> Influenza A virus
<400> 280
   Pro Asn Asp Ala Ala Glu Gln Thr Arg Leu Tyr Gln Asn Ser Asn Thr
   1 5 10 15
   Tyr Gly
<210> 281
   <211> 18
   <212> PRT
   <213> Influenza A virus
<400> 281
   Pro Asn Asp Ala Ala Glu Gln Ile Lys Leu Tyr Gln Asn Pro Thr Thr
   1 5 10 15
   Tyr Gly
<210> 282
   <211> 18
   <212> PRT
   <213> Influenza A virus
<400> 282
   Pro Asn Asp Ala Ala Glu Gln Ile Lys Leu Tyr Gln Asn Pro Asn Thr
   1 5 10 15
   Tyr Gly
<210> 283
   <211> 18
   <212> PRT
   <213> Influenza A virus
<400> 283
   Pro Asn Asp Ala Ala Glu Gln Ile Lys Leu Tyr Gln Asn Ser Thr Thr
   1 5 10 15
   Tyr Gly
<210> 284
   <211> 18
   <212> PRT
   <213> Influenza A virus
<400> 284
   Pro Asn Asp Ala Ala Glu Gln Ile Lys Leu Tyr Gln Asn Ser Asn Thr
   1 5 10 15
   Tyr Gly
<210> 285
   <211> 18
   <212> PRT
   <213> Influenza A virus
<400> 285
   Pro Asn Asp Ala Ala Glu Gln Ile Arg Leu Tyr Gln Asn Pro Thr Thr
   1 5 10 15
   Tyr Gly
<210> 286
   <211> 18
   <212> PRT
   <213> Influenza A virus
<400> 286
   Pro Asn Asp Ala Ala Glu Gln Ile Arg Leu Tyr Gln Asn Pro Asn Thr
   1 5 10 15
   Tyr Gly
<210> 287
   <211> 18
   <212> PRT
   <213> Influenza A virus
<400> 287
   Pro Asn Asp Ala Ala Glu Gln Ile Arg Leu Tyr Gln Asn Ser Thr Thr
   1 5 10 15
   Tyr Gly
<210> 288
   <211> 18
   <212> PRT
   <213> Influenza A virus
<400> 288
   Pro Asn Asp Ala Ala Glu Gln Ile Arg Leu Tyr Gln Asn Ser Asn Thr
   1               5                   10                  15
   Tyr Gly
<210> 289
   <211> 19
   <212> PRT
   <213> Influenza A virus
<400> 289
   Ser Trp Ser Asn His Glu Ala Ser Leu Ile Lys Lys Asn Ser Ala Tyr
   1               5                   10                  15
   Pro Thr Gly
<210> 290
   <211> 19
   <212> PRT
   <213> Influenza A virus
<400> 290
   Ser Trp Ser Asn His Glu Ala Ser Leu Ile Lys Lys Asn Ser Thr Tyr
   1               5                   10                  15
   Pro Thr Gly
<210> 291
   <211> 19
   <212> PRT
   <213> Influenza A virus
<400> 291
   Ser Trp Ser Asn His Glu Ala Ser Leu Ile Lys Lys Asn Ser Val Tyr
   1               5                   10                  15
   Pro Thr Gly
<210> 292
   <211> 19
   <212> PRT
   <213> Influenza A virus
<400> 292
   Ser Trp Ser Asn His Glu Ala Ser Leu Ile Lys Lys Asn Asn Ala Tyr
   1               5                   10                  15
   Pro Thr Gly
<210> 293
   <211> 19
   <212> PRT
   <213> Influenza A virus
<400> 293
   Ser Trp Ser Asn His Glu Ala Ser Leu Ile Lys Lys Asn Asn Thr Tyr
   1               5                   10                  15
   Pro Thr Gly
<210> 294
   <211> 19
   <212> PRT
   <213> Influenza A virus
<400> 294
   Ser Trp Ser Asn His Glu Ala Ser Leu Ile Lys Lys Asn Asn Val Tyr
   1               5                   10                  15
   Pro Thr Gly
<210> 295
   <211> 19
   <212> PRT
   <213> Influenza A virus
<400> 295
   Ser Trp Ser Asn His Asp Ala Ser Leu Ile Lys Lys Asn Ser Ala Tyr
   1               5                   10                  15
   Pro Thr Gly
<210> 296
   <211> 19
   <212> PRT
   <213> Influenza A virus
<400> 296
   Ser Trp Ser Asn His Asp Ala Ser Leu Ile Lys Lys Asn Ser Thr Tyr
   1               5                   10                  15
   Pro Thr Gly
<210> 297
   <211> 19
   <212> PRT
   <213> Influenza A virus
<400> 297
   Ser Trp Ser Asn His Asp Ala Ser Leu Ile Lys Lys Asn Ser Val Tyr
   1 5 10 15
   Pro Thr Gly
<210> 298
   <211> 19
   <212> PRT
   <213> Influenza A virus
<400> 298
   Ser Trp Ser Asn His Asp Ala Ser Leu Ile Lys Lys Asn Asn Ala Tyr
   1 5 10 15
   Pro Thr Gly
<210> 299
   <211> 19
   <212> PRT
   <213> Influenza A virus
<400> 299
   Ser Trp Ser Asn His Asp Ala Ser Leu Ile Lys Lys Asn Asn Thr Tyr
   1 5 10 15
   Pro Thr Gly
<210> 300
   <211> 19
   <212> PRT
   <213> Influenza A virus
<400> 300
   Ser Trp Ser Asn His Asp Ala Ser Leu Ile Lys Lys Asn Asn Val Tyr
   1 5 10 15
   Pro Thr Gly
<210> 301
   <211> 19
   <212> PRT
   <213> Influenza A virus
<400> 301
   Ser Trp Ser Ser His Glu Ala Ser Leu Ile Lys Lys Asn Ser Ala Tyr
   1 5 10 15
   Pro Thr Gly
<210> 302
   <211> 19
   <212> PRT
   <213> Influenza A virus
<400> 302
   Ser Trp Ser Ser His Glu Ala Ser Leu Ile Lys Lys Asn Ser Thr Tyr
   1 5 10 15
   Pro Thr Gly
<210> 303
   <211> 19
   <212> PRT
   <213> Influenza A virus
<400> 303
   Ser Trp Ser Ser His Glu Ala Ser Leu Ile Lys Lys Asn Ser Val Tyr
   1 5 10 15
   Pro Thr Gly
<210> 304
   <211> 19
   <212> PRT
   <213> Influenza A virus
<400> 304
   Ser Trp Ser Ser His Glu Ala Ser Leu Ile Lys Lys Asn Asn Ala Tyr
   1 5 10 15
   Pro Thr Gly
<210> 305
   <211> 19
   <212> PRT
   <213> Influenza A virus
<400> 305
   Ser Trp Ser Ser His Glu Ala Ser Leu Ile Lys Lys Asn Asn Thr Tyr
   1 5 10 15
   Pro Thr Gly
<210> 306
   <211> 19
   <212> PRT
   <213> Influenza A virus
<400> 306
   Ser Trp Ser Ser His Glu Ala Ser Leu Ile Lys Lys Asn Asn Val Tyr
   1 5 10 15
   Pro Thr Gly
<210> 307
   <211> 19
   <212> PRT
   <213> Influenza A virus
<400> 307
   Ser Trp Ser Ser His Asp Ala Ser Leu Ile Lys Lys Asn Ser Ala Tyr
   1 5 10 15
   Pro Thr Gly
<210> 308
   <211> 19
   <212> PRT
   <213> Influenza A virus
<400> 308
   Ser Trp Ser Ser His Asp Ala Ser Leu Ile Lys Lys Asn Ser Thr Tyr
   1 5 10 15
   Pro Thr Gly
<210> 309
   <211> 19
   <212> PRT
   <213> Influenza A virus
<400> 309
   Ser Trp Ser Ser His Asp Ala Ser Leu Ile Lys Lys Asn Ser Val Tyr
   1 5 10 15
   Pro Thr Gly
<210> 310
   <211> 19
   <212> PRT
   <213> Influenza A virus
<400> 310
   Ser Trp Ser Ser His Asp Ala Ser Leu Ile Lys Lys Asn Asn Ala Tyr
   1 5 10 15
   Pro Thr Gly
<210> 311
   <211> 19
   <212> PRT
   <213> Influenza A virus
<400> 311
   Ser Trp Ser Ser His Asp Ala Ser Leu Ile Lys Lys Asn Asn Thr Tyr
   1 5 10 15
   Pro Thr Gly
<210> 312
   <211> 19
   <212> PRT
   <213> Influenza A virus
<400> 312
   Ser Trp Ser Ser His Asp Ala Ser Leu Ile Lys Lys Asn Asn Val Tyr
   1 5 10 15
   Pro Thr Gly
<210> 313
   <211> 24
   <212> PRT
   <213> Influenza A virus
<400> 313
   Gly Lys Leu Ser Asp Leu Asp Gly Val Lys Pro Leu Ile Leu Leu Glu
   1 5 10 15
   Tyr Gly Asn Ser Asn Thr Lys Gly
   20
<210> 314
   <211> 24
   <212> PRT
   <213> Influenza A virus
<400> 314
   Gly Lys Leu Ser Asp Leu Lys Gly Val Lys Pro Leu Ile Leu Leu Glu
   1 5 10 15
   Tyr Gly Asn Ser Asn Thr Lys Gly
   20
<210> 315
   <211> 24
   <212> PRT
   <213> Influenza A virus
<400> 315
   Gly Lys Leu Ser Asp Leu Asn Gly Val Lys Pro Leu Ile Leu Leu Glu
   1               5                   10                  15
   Tyr Gly Asn Ser Asn Thr Lys Gly
               20
<210> 316
   <211> 24
   <212> PRT
   <213> Influenza A virus
<400> 316
   Gly Lys Leu Ser Asp Leu Asp Gly Val Lys Pro Leu Ile Leu Leu Glu
   1               5                   10                  15
   Tyr Gly Asn Ser Asn Ala Lys Gly
               20
<210> 317
   <211> 24
   <212> PRT
   <213> Influenza A virus
<400> 317
   Gly Lys Leu Ser Asp Leu Lys Gly Val Lys Pro Leu Ile Leu Leu Glu
   1               5                   10                  15
   Tyr Gly Asn Ser Asn Ala Lys Gly
               20
<210> 318
   <211> 24
   <212> PRT
   <213> Influenza A virus
<400> 318
   Gly Lys Leu Ser Asp Leu Asn Gly Val Lys Pro Leu Ile Leu Leu Glu
   1               5                   10                  15
   Tyr Gly Asn Ser Asn Ala Lys Gly
               20
<210> 319
   <211> 24
   <212> PRT
   <213> Influenza A virus
<400> 319
   Gly Lys Leu Ser Asp Leu Asp Gly Val Lys Pro Leu Ile Leu Leu Glu
   1 5 10 15
   Tyr Gly Asn Ser Asp Thr Lys Gly
   20
<210> 320
   <211> 24
   <212> PRT
   <213> Influenza A virus
<400> 320
   Gly Lys Leu Ser Asp Leu Lys Gly Val Lys Pro Leu Ile Leu Leu Glu
   1 5 10 15
   Tyr Gly Asn Ser Asp Thr Lys Gly
   20
<210> 321
   <211> 24
   <212> PRT
   <213> Influenza A virus
<400> 321
   Gly Lys Leu Ser Asp Leu Asn Gly Val Lys Pro Leu Ile Leu Leu Glu
   1 5 10 15
   Tyr Gly Asn Ser Asp Thr Lys Gly
   20
<210> 322
   <211> 24
   <212> PRT
   <213> Influenza A virus
<400> 322
   Gly Lys Leu Ser Asp Leu Asp Gly Val Lys Pro Leu Ile Leu Leu Glu
   1 5 10 15
   Tyr Gly Asn Ser Asp Ala Lys Gly
   20
<210> 323
   <211> 24
   <212> PRT
   <213> Influenza A virus
<400> 323
   Gly Lys Leu Ser Asp Leu Lys Gly Val Lys Pro Leu Ile Leu Leu Glu
   1 5 10 15
   Tyr Gly Asn Ser Asp Ala Lys Gly
   20
<210> 324
   <211> 24
   <212> PRT
   <213> Influenza A virus
<400> 324
   Gly Lys Leu Ser Asp Leu Asn Gly Val Lys Pro Leu Ile Leu Leu Glu
   1               5                   10                  15
   Tyr Gly Asn Ser Asp Ala Lys Gly
               20
<210> 325
   <211> 24
   <212> PRT
   <213> Influenza A virus
<400> 325
   Gly Lys Leu Ser Ser Leu Asp Gly Val Lys Pro Leu Ile Leu Leu Glu
   1               5                   10                  15
   Tyr Gly Asn Ser Asn Thr Lys Gly
               20
<210> 326
   <211> 24
   <212> PRT
   <213> Influenza A virus
<400> 326
   Gly Lys Leu Ser Ser Leu Lys Gly Val Lys Pro Leu Ile Leu Leu Glu
   1               5                   10                  15
   Tyr Gly Asn Ser Asn Thr Lys Gly
               20
<210> 327
   <211> 24
   <212> PRT
   <213> Influenza A virus
<400> 327
   Gly Lys Leu Ser Ser Leu Asn Gly Val Lys Pro Leu Ile Leu Leu Glu
   1               5                   10                  15
   Tyr Gly Asn Ser Asn Thr Lys Gly
               20

<210> 328
   <211> 24
   <212> PRT
   <213> Influenza A virus
<400> 328
   Gly Lys Leu Ser Ser Leu Asp Gly Val Lys Pro Leu Ile Leu Leu Glu
   1               5                   10                  15
   Tyr Gly Asn Ser Asn Ala Lys Gly
               20
<210> 329
   <211> 24
   <212> PRT
   <213> Influenza A virus
<400> 329
   Gly Lys Leu Ser Ser Leu Lys Gly Val Lys Pro Leu Ile Leu Leu Glu
   1               5                   10                  15
   Tyr Gly Asn Ser Asn Ala Lys Gly
               20
<210> 330
   <211> 24
   <212> PRT
   <213> Influenza A virus
<400> 330
   Gly Lys Leu Ser Ser Leu Asn Gly Val Lys Pro Leu Ile Leu Leu Glu
   1               5                   10                  15
   Tyr Gly Asn Ser Asn Ala Lys Gly
               20
<210> 331
   <211> 24
   <212> PRT
   <213> Influenza A virus
<400> 331
   Gly Lys Leu Ser Ser Leu Asp Gly Val Lys Pro Leu Ile Leu Leu Glu
   1               5                   10                  15
   Tyr Gly Asn Ser Asp Thr Lys Gly
               20
<210> 332
   <211> 24
   <212> PRT
   <213> Influenza A virus
<400> 332
   Gly Lys Leu Ser Ser Leu Lys Gly Val Lys Pro Leu Ile Leu Leu Glu
   1 5 10 15
   Tyr Gly Asn Ser Asp Thr Lys Gly
   20
<210> 333
   <211> 24
   <212> PRT
   <213> Influenza A virus
<400> 333
   Gly Lys Leu Ser Ser Leu Asn Gly Val Lys Pro Leu Ile Leu Leu Glu
   1 5 10 15
   Tyr Gly Asn Ser Asp Thr Lys Gly
   20
<210> 334
   <211> 24
   <212> PRT
   <213> Influenza A virus
<400> 334
   Gly Lys Leu Ser Ser Leu Asp Gly Val Lys Pro Leu Ile Leu Leu Glu
   1 5 10 15
   Tyr Gly Asn Ser Asp Ala Lys Gly
   20
<210> 335
   <211> 24
   <212> PRT
   <213> Influenza A virus
<400> 335
   Gly Lys Leu Ser Ser Leu Lys Gly Val Lys Pro Leu Ile Leu Leu Glu
   1 5 10 15
   Tyr Gly Asn Ser Asp Ala Lys Gly
   20
<210> 336
   <211> 24
   <212> PRT
   <213> Influenza A virus
<400> 336
   Gly Lys Leu Ser Ser Leu Asn Gly Val Lys Pro Leu Ile Leu Leu Glu
   1 5 10 15
   Tyr Gly Asn Ser Asp Ala Lys Gly
   20
<210> 337
   <211> 25
   <212> PRT
   <213> Influenza A virus
<400> 337
   Thr Ile Lys Arg Ser Tyr Asn Asn Thr Asn Gln Glu Asp Lys Pro Asn
   1 5 10 15
   Asp Ala Ile Asn Phe Glu Ser Asn Gly
   20 25
<210> 338
   <211> 25
   <212> PRT
   <213> Influenza A virus
<400> 338
   Thr Ile Lys Arg Ser Tyr Asn Asn Thr Asn Gln Glu Asp Lys Pro Asn
   1 5 10 15
   Asp Ala Ile Asn Phe Glu Ser Thr Gly
   20 25
<210> 339
   <211> 25
   <212> PRT
   <213> Influenza A virus
<400> 339
   Thr Ile Lys Arg Ser Tyr Asn Asn Thr Asn Gln Glu Asp Lys Pro Asn
   1 5 10 15
   Asp Ser Ile Asn Phe Glu Ser Asn Gly
   20 25
<210> 340
   <211> 25
   <212> PRT
   <213> Influenza A virus
<400> 340
   Thr Ile Lys Arg Ser Tyr Asn Asn Thr Asn Gln Glu Asp Lys Pro Asn
   1 5 10 15
   Asp Ser Ile Asn Phe Glu Ser Thr Gly
   20 25
<210> 341
   <211> 25
   <212> PRT
   <213> Influenza A virus
<400> 341
   Thr Ile Lys Arg Ser Tyr Asn Asn Thr Asn Gln Glu Asp Arg Pro Asn
   1               5                   10                  15
   Asp Ala Ile Asn Phe Glu Ser Asn Gly
               20                  25
<210> 342
   <211> 25
   <212> PRT
   <213> Influenza A virus
<400> 342
   Thr Ile Lys Arg Ser Tyr Asn Asn Thr Asn Gln Glu Asp Arg Pro Asn
   1               5                   10                  15
   Asp Ala Ile Asn Phe Glu Ser Thr Gly
               20                  25
<210> 343
   <211> 25
   <212> PRT
   <213> Influenza A virus
<400> 343
   Thr Ile Lys Arg Ser Tyr Asn Asn Thr Asn Gln Glu Asp Arg Pro Asn
   1               5                   10                  15
   Asp Ser Ile Asn Phe Glu Ser Asn Gly
               20                  25
<210> 344
   <211> 25
   <212> PRT
   <213> Influenza A virus
<400> 344
   Thr Ile Lys Arg Ser Tyr Asn Asn Thr Asn Gln Glu Asp Arg Pro Asn
   1               5                   10                  15
   Asp Ser Ile Asn Phe Glu Ser Thr Gly
               20                  25
<210> 345
   <211> 25
   <212> PRT
   <213> Influenza A virus
<400> 345
   Thr Ile Lys Arg Ser Tyr Asn Asn Thr Asn Val Glu Asp Lys Pro Asn
   1               5                   10                  15
   Asp Ala Ile Asn Phe Glu Ser Asn Gly
               20                  25
<210> 346
   <211> 25
   <212> PRT
   <213> Influenza A virus
<400> 346
   Thr Ile Lys Arg Ser Tyr Asn Asn Thr Asn Val Glu Asp Lys Pro Asn
   1               5                   10                  15
   Asp Ala Ile Asn Phe Glu Ser Thr Gly
               20                  25
<210> 347
   <211> 25
   <212> PRT
   <213> Influenza A virus
<400> 347
   Thr Ile Lys Arg Ser Tyr Asn Asn Thr Asn Val Glu Asp Lys Pro Asn
   1               5                   10                  15
   Asp Ser Ile Asn Phe Glu Ser Asn Gly
               20                  25
<210> 348
   <211> 25
   <212> PRT
   <213> Influenza A virus
<400> 348
   Thr Ile Lys Arg Ser Tyr Asn Asn Thr Asn Val Glu Asp Lys Pro Asn
   1               5                   10                  15
   Asp Ser Ile Asn Phe Glu Ser Thr Gly
               20                  25
<210> 349
   <211> 25
   <212> PRT
   <213> Influenza A virus
<400> 349
   Thr Ile Lys Arg Ser Tyr Asn Asn Thr Asn Val Glu Asp Arg Pro Asn
   1               5                   10                  15
   Asp Ala Ile Asn Phe Glu Ser Asn Gly
               20                  25
<210> 350
   <211> 25
   <212> PRT
   <213> Influenza A virus
<400> 350
   Thr Ile Lys Arg Ser Tyr Asn Asn Thr Asn Val Glu Asp Arg Pro Asn
   1               5                   10                  15
   Asp Ala Ile Asn Phe Glu Ser Thr Gly
               20                  25
<210> 351
   <211> 25
   <212> PRT
   <213> Influenza A virus
<400> 351
   Thr Ile Lys Arg Ser Tyr Asn Asn Thr Asn Val Glu Asp Arg Pro Asn
   1               5                   10                  15
   Asp Ser Ile Asn Phe Glu Ser Asn Gly
               20                  25
<210> 352
   <211> 25
   <212> PRT
   <213> Influenza A virus
<400> 352
   Thr Ile Lys Arg Ser Tyr Asn Asn Thr Asn Val Glu Asp Arg Pro Asn
   1               5                   10                  15
   Asp Ser Ile Asn Phe Glu Ser Thr Gly
               20                  25
<210> 353
   <211> 25
   <212> PRT
   <213> Influenza A virus
<400> 353
   Thr Ile Lys Arg Thr Tyr Asn Asn Thr Asn Gln Glu Asp Lys Pro Asn
   1               5                   10                  15
   Asp Ala Ile Asn Phe Glu Ser Asn Gly
               20                  25
<210> 354
   <211> 25
   <212> PRT
   <213> Influenza A virus
<400> 354
   Thr Ile Lys Arg Thr Tyr Asn Asn Thr Asn Gln Glu Asp Lys Pro Asn
   1 5 10 15
   Asp Ala Ile Asn Phe Glu Ser Thr Gly
   20 25
<210> 355
   <211> 25
   <212> PRT
   <213> Influenza A virus
<400> 355
   Thr Ile Lys Arg Thr Tyr Asn Asn Thr Asn Gln Glu Asp Lys Pro Asn
   1 5 10 15
   Asp Ser Ile Asn Phe Glu Ser Asn Gly
   20 25
<210> 356
   <211> 25
   <212> PRT
   <213> Influenza A virus
<400> 356
   Thr Ile Lys Arg Thr Tyr Asn Asn Thr Asn Gln Glu Asp Lys Pro Asn
   1 5 10 15
   Asp Ser Ile Asn Phe Glu Ser Thr Gly
   20 25
<210> 357
   <211> 25
   <212> PRT
   <213> Influenza A virus
<400> 357
   Thr Ile Lys Arg Thr Tyr Asn Asn Thr Asn Gln Glu Asp Arg Pro Asn
   1 5 10 15
   Asp Ala Ile Asn Phe Glu Ser Asn Gly
   20 25
<210> 358
   <211> 25
   <212> PRT
   <213> Influenza A virus
<400> 358
   Thr Ile Lys Arg Thr Tyr Asn Asn Thr Asn Gln Glu Asp Arg Pro Asn
   1 5 10 15
   Asp Ala Ile Asn Phe Glu Ser Thr Gly
   20 25
<210> 359
   <211> 25
   <212> PRT
   <213> Influenza A virus
<400> 359
   Thr Ile Lys Arg Thr Tyr Asn Asn Thr Asn Gln Glu Asp Arg Pro Asn
   1               5                   10                  15
   Asp Ser Ile Asn Phe Glu Ser Asn Gly
               20                  25
<210> 360
   <211> 25
   <212> PRT
   <213> Influenza A virus
<400> 360
   Thr Ile Lys Arg Thr Tyr Asn Asn Thr Asn Gln Glu Asp Arg Pro Asn
   1               5                   10                  15
   Asp Ser Ile Asn Phe Glu Ser Thr Gly
               20                  25
<210> 361
   <211> 25
   <212> PRT
   <213> Influenza A virus
<400> 361
   Thr Ile Lys Arg Thr Tyr Asn Asn Thr Asn Val Glu Asp Lys Pro Asn
   1               5                   10                  15
   Asp Ala Ile Asn Phe Glu Ser Asn Gly
               20                  25
<210> 362
   <211> 25
   <212> PRT
   <213> Influenza A virus
<400> 362
   Thr Ile Lys Arg Thr Tyr Asn Asn Thr Asn Val Glu Asp Lys Pro Asn
   1               5                   10                  15
   Asp Ala Ile Asn Phe Glu Ser Thr Gly
               20                  25
<210> 363
   <211> 25
   <212> PRT
   <213> Influenza A virus
<400> 363
   Thr Ile Lys Arg Thr Tyr Asn Asn Thr Asn Val Glu Asp Lys Pro Asn
   1 5 10 15
   Asp Ser Ile Asn Phe Glu Ser Asn Gly
   20 25
<210> 364
   <211> 25
   <212> PRT
   <213> Influenza A virus
<400> 364
   Thr Ile Lys Arg Thr Tyr Asn Asn Thr Asn Val Glu Asp Lys Pro Asn
   1 5 10 15
   Asp Ser Ile Asn Phe Glu Ser Thr Gly
   20 25
<210> 365
   <211> 25
   <212> PRT
   <213> Influenza A virus
<400> 365
   Thr Ile Lys Arg Thr Tyr Asn Asn Thr Asn Val Glu Asp Arg Pro Asn
   1 5 10 15
   Asp Ala Ile Asn Phe Glu Ser Asn Gly
   20 25
<210> 366
   <211> 25
   <212> PRT
   <213> Influenza A virus
<400> 366
   Thr Ile Lys Arg Thr Tyr Asn Asn Thr Asn Val Glu Asp Arg Pro Asn
   1 5 10 15
   Asp Ala Ile Asn Phe Glu Ser Thr Gly
   20 25
<210> 367
   <211> 25
   <212> PRT
   <213> Influenza A virus
<400> 367
   Thr Ile Lys Arg Thr Tyr Asn Asn Thr Asn Val Glu Asp Arg Pro Asn
   1 5 10 15
   Asp Ser Ile Asn Phe Glu Ser Asn Gly
   20 25
<210> 368
   <211> 25
   <212> PRT
   <213> Influenza A virus
<400> 368
   Thr Ile Lys Arg Thr Tyr Asn Asn Thr Asn Val Glu Asp Arg Pro Asn
   1 5 10 15
   Asp Ser Ile Asn Phe Glu Ser Thr Gly
   20 25
<210> 369
   <211> 17
   <212> PRT
   <213> Influenza A virus
<400> 369
   Ile Ser Val Gly Thr Ser Thr Leu Asn Gln Arg Leu Val Pro Lys Ile
   1 5 10 15
   Gly
<210> 370
   <211> 17
   <212> PRT
   <213> Influenza A virus
<400> 370
   Ile Ser Val Gly Thr Ser Thr Leu Asn Gln Arg Leu Val Pro Glu Ile
   1 5 10 15
   Gly
<210> 371
   <211> 17
   <212> PRT
   <213> Influenza A virus
<400> 371
   Ile Ser Val Gly Thr Ser Thr Leu Asn Gln Arg Leu Val Pro Arg Ile
   1 5 10 15
   Gly
<210> 372
   <211> 17
   <212> PRT
   <213> Influenza A virus
<400> 372
   Ile Ser Val Gly Thr Ser Thr Leu Asn Gln Arg Leu Ile Pro Lys Ile
   1 5 10 15
   Gly
<210> 373
   <211> 17
   <212> PRT
   <213> Influenza A virus
<400> 373
   Ile Ser Val Gly Thr Ser Thr Leu Asn Gln Arg Leu Ile Pro Glu Ile
   1 5 10 15
   Gly
<210> 374
   <211> 17
   <212> PRT
   <213> Influenza A virus
<400> 374
   Ile Ser Val Gly Thr Ser Thr Leu Asn Gln Arg Leu Ile Pro Arg Ile
   1 5 10 15
   Gly
<210> 375
   <211> 17
   <212> PRT
   <213> Influenza A virus
<400> 375
   Ile Ser Val Gly Thr Ser Thr Leu Asn Gln Arg Ser Val Pro Lys Ile
   1 5 10 15
   Gly
<210> 376
   <211> 17
   <212> PRT
   <213> Influenza A virus
<400> 376
   Ile Ser Val Gly Thr Ser Thr Leu Asn Gln Arg Ser Val Pro Glu Ile
   1 5 10 15
   Gly
<210> 377
   <211> 17
   <212> PRT
   <213> Influenza A virus
<400> 377
   Ile Ser Val Gly Thr Ser Thr Leu Asn Gln Arg Ser Val Pro Arg Ile
   1 5 10 15
   Gly
<210> 378
   <211> 17
   <212> PRT
   <213> Influenza A virus
<400> 378
   Ile Ser Val Gly Thr Ser Thr Leu Asn Gln Arg Ser Ile Pro Lys Ile
   1 5 10 15
   Gly
<210> 379
   <211> 17
   <212> PRT
   <213> Influenza A virus
<400> 379
   Ile Ser Val Gly Thr Ser Thr Leu Asn Gln Arg Ser Ile Pro Glu Ile
   1 5 10 15
   Gly
<210> 380
   <211> 17
   <212> PRT
   <213> Influenza A virus
<400> 380
   Ile Ser Val Gly Thr Ser Thr Leu Asn Gln Arg Ser Ile Pro Arg Ile
   1 5 10 15
   Gly
<210> 381
   <211> 17
   <212> PRT
   <213> Influenza A virus
<400> 381
   Val Ser Val Gly Thr Ser Thr Leu Asn Gln Arg Leu Val Pro Lys Ile
   1 5 10 15
   Gly
<210> 382
   <211> 17
   <212> PRT
   <213> Influenza A virus
<400> 382
   Val Ser Val Gly Thr Ser Thr Leu Asn Gln Arg Leu Val Pro Glu Ile
   1 5 10 15
   Gly
<210> 383
   <211> 17
   <212> PRT
   <213> Influenza A virus
<400> 383
   Val Ser Val Gly Thr Ser Thr Leu Asn Gln Arg Leu Val Pro Arg Ile
   1 5 10 15
   Gly
<210> 384
   <211> 17
   <212> PRT
   <213> Influenza A virus
<400> 384
   Val Ser Val Gly Thr Ser Thr Leu Asn Gln Arg Leu Ile Pro Lys Ile
   1 5 10 15
   Gly
<210> 385
   <211> 17
   <212> PRT
   <213> Influenza A virus
<400> 385
   Val Ser Val Gly Thr Ser Thr Leu Asn Gln Arg Leu Ile Pro Glu Ile
   1               5                   10                  15
   Gly
<210> 386
   <211> 17
   <212> PRT
   <213> Influenza A virus
<400> 386
   Val Ser Val Gly Thr Ser Thr Leu Asn Gln Arg Leu Ile Pro Arg Ile
   1               5                   10                  15
   Gly
<210> 387
   <211> 17
   <212> PRT
   <213> Influenza A virus
<400> 387
   Val Ser Val Gly Thr Ser Thr Leu Asn Gln Arg Ser Val Pro Lys Ile
   1               5                   10                  15
   Gly
<210> 388
   <211> 17
   <212> PRT
   <213> Influenza A virus
<400> 388
   Val Ser Val Gly Thr Ser Thr Leu Asn Gln Arg Ser Val Pro Glu Ile
   1               5                   10                  15
   Gly
<210> 389
   <211> 17
   <212> PRT
   <213> Influenza A virus
<400> 389
   Val Ser Val Gly Thr Ser Thr Leu Asn Gln Arg Ser Val Pro Arg Ile
   1               5                   10                  15
   Gly
<210> 390
   <211> 17
   <212> PRT
   <213> Influenza A virus
<400> 390
   Val Ser Val Gly Thr Ser Thr Leu Asn Gln Arg Ser Ile Pro Lys Ile
   1 5 10 15
   Gly
<210> 391
   <211> 17
   <212> PRT
   <213> Influenza A virus
<400> 391
   Val Ser Val Gly Thr Ser Thr Leu Asn Gln Arg Ser Ile Pro Glu Ile
   1 5 10 15
   Gly
<210> 392
   <211> 17
   <212> PRT
   <213> Influenza A virus
<400> 392
   Val Ser Val Gly Thr Ser Thr Leu Asn Gln Arg Ser Ile Pro Arg Ile
   1 5 10 15
   Gly
<210> 393
   <211> 23
   <212> PRT
   <213> Influenza A virus
<400> 393
   Lys Ala Asn Pro Ala Asn Asp Leu Gly Asn Pro Met Ser Asp Glu Phe
   1 5 10 15
   Ile Asn Val Pro Glu Trp Gly
   20
<210> 394
   <211> 23
   <212> PRT
   <213> Influenza A virus
<400> 394
   Lys Ala Asn Pro Ala Asn Asp Leu Gly Asn Pro Met Ser Asp Glu Phe
   1               5                   10                  15
   Leu Asn Val Pro Glu Trp Gly
               20
<210> 395
   <211> 23
   <212> PRT
   <213> Influenza A virus
<400> 395
   Lys Ala Asn Pro Ala Asn Gly Leu Gly Asn Pro Met Ser Asp Glu Phe
   1               5                   10                  15
   Ile Asn Val Pro Glu Trp Gly
                                       20
<210> 396
   <211> 23
   <212> PRT
   <213> Influenza A virus
<400> 396
   Lys Ala Asn Pro Ala Asn Gly Leu Gly Asn Pro Met Ser Asp Glu Phe
   1               5                   10                  15
   Leu Asn Val Pro Glu Trp Gly
               20
<210> 397
   <211> 23
   <212> PRT
   <213> Influenza A virus
<400> 397
   Lys Ala Asn Pro Val Asn Asp Leu Gly Asn Pro Met Ser Asp Glu Phe
   1               5                   10                  15
   Ile Asn Val Pro Glu Trp Gly
               20
<210> 398
   <211> 23
   <212> PRT
   <213> Influenza A virus
<400> 398
   Lys Ala Asn Pro Val Asn Asp Leu Gly Asn Pro Met Ser Asp Glu Phe
   1               5                   10                  15
   Leu Asn Val Pro Glu Trp Gly
               20
<210> 399
   <211> 23
   <212> PRT
   <213> Influenza A virus
<400> 399
   Lys Ala Asn Pro Val Asn Gly Leu Gly Asn Pro Met Ser Asp Glu Phe
   1               5                   10                  15
   Ile Asn Val Pro Glu Trp Gly
               20
<210> 400
   <211> 23
   <212> PRT
   <213> Influenza A virus
<400> 400
   Lys Ala Asn Pro Val Asn Gly Leu Gly Asn Pro Met Ser Asp Glu Phe
   1               5                   10                  15
   Leu Asn Val Pro Glu Trp Gly
               20
<210> 401
   <211> 23
   <212> PRT
   <213> Influenza A virus
<400> 401
   Lys Ala Ser Pro Ala Asn Asp Leu Gly Asn Pro Met Ser Asp Glu Phe
   1               5                   10                  15
   Ile Asn Val Pro Glu Trp Gly
               20
<210> 402
   <211> 23
   <212> PRT
   <213> Influenza A virus
<400> 402
   Lys Ala Ser Pro Ala Asn Asp Leu Gly Asn Pro Met Ser Asp Glu Phe
   1               5                   10                  15
   Leu Asn Val Pro Glu Trp Gly
               20
<210> 403
   <211> 23
   <212> PRT
   <213> Influenza A virus
<400> 403
   Lys Ala Ser Pro Ala Asn Gly Leu Gly Asn Pro Met Ser Asp Glu Phe
   1               5                   10                  15
   Ile Asn Val Pro Glu Trp Gly
               20
<210> 404
   <211> 23
   <212> PRT
   <213> Influenza A virus
<400> 404
   Lys Ala Ser Pro Ala Asn Gly Leu Gly Asn Pro Met Ser Asp Glu Phe
   1               5                   10                  15
   Leu Asn Val Pro Glu Trp Gly
               20
<210> 405
   <211> 23
   <212> PRT
   <213> Influenza A virus
<400> 405
   Lys Ala Ser Pro Val Asn Asp Leu Gly Asn Pro Met Ser Asp Glu Phe
   1               5                   10                  15
   Ile Asn Val Pro Glu Trp Gly
               20
<210> 406
   <211> 23
   <212> PRT
   <213> Influenza A virus
<400> 406
   Lys Ala Ser Pro Val Asn Asp Leu Gly Asn Pro Met Ser Asp Glu Phe
   1               5                   10                  15
   Leu Asn Val Pro Glu Trp Gly
               20
<210> 407
   <211> 23
   <212> PRT
   <213> Influenza A virus
<400> 407
   Lys Ala Ser Pro Val Asn Gly Leu Gly Asn Pro Met Ser Asp Glu Phe
   1 5 10 15
   Ile Asn Val Pro Glu Trp Gly
   20
<210> 408
   <211> 23
   <212> PRT
   <213> Influenza A virus
<400> 408
   Lys Ala Ser Pro Val Asn Gly Leu Gly Asn Pro Met Ser Asp Glu Phe
   1 5 10 15
   Leu Asn Val Pro Glu Trp Gly
   20
<210> 409
   <211> 23
   <212> PRT
   <213> Influenza A virus
<400> 409
   Lys Asp Asn Pro Ala Asn Asp Leu Gly Asn Pro Met Ser Asp Glu Phe
   1 5 10 15
   Ile Asn Val Pro Glu Trp Gly
   20
<210> 410
   <211> 23
   <212> PRT
   <213> Influenza A virus
<400> 410
   Lys Asp Asn Pro Ala Asn Asp Leu Gly Asn Pro Met Ser Asp Glu Phe
   1 5 10 15
   Leu Asn Val Pro Glu Trp Gly
   20
<210> 411
   <211> 23
   <212> PRT
   <213> Influenza A virus
<400> 411
   Lys Asp Asn Pro Ala Asn Gly Leu Gly Asn Pro Met Ser Asp Glu Phe
   1 5 10 15
   Ile Asn Val Pro Glu Trp Gly
   20
<210> 412
   <211> 23
   <212> PRT
   <213> Influenza A virus
<400> 412
   Lys Asp Asn Pro Ala Asn Gly Leu Gly Asn Pro Met Ser Asp Glu Phe
   1               5                   10                  15
   Leu Asn Val Pro Glu Trp Gly
               20

<210> 413
   <211> 23
   <212> PRT
   <213> Influenza A virus
<400> 413
   Lys Asp Asn Pro Val Asn Asp Leu Gly Asn Pro Met Ser Asp Glu Phe
   1               5                   10                  15
   Ile Asn Val Pro Glu Trp Gly
               20
<210> 414
   <211> 23
   <212> PRT
   <213> Influenza A virus
<400> 414
   Lys Asp Asn Pro Val Asn Asp Leu Gly Asn Pro Met Ser Asp Glu Phe
   1               5                   10                  15
   Leu Asn Val Pro Glu Trp Gly
               20
<210> 415
   <211> 23
   <212> PRT
   <213> Influenza A virus
<400> 415
   Lys Asp Asn Pro Val Asn Gly Leu Gly Asn Pro Met Ser Asp Glu Phe
   1               5                   10                  15
   Ile Asn Val Pro Glu Trp Gly
               20
<210> 416
   <211> 23
   <212> PRT
   <213> Influenza A virus
<400> 416
   Lys Asp Asn Pro Val Asn Gly Leu Gly Asn Pro Met Ser Asp Glu Phe
   1               5                   10                  15
   Leu Asn Val Pro Glu Trp Gly
               20
<210> 417
   <211> 23
   <212> PRT
   <213> Influenza A virus
<400> 417
   Lys Asp Ser Pro Ala Asn Asp Leu Gly Asn Pro Met Ser Asp Glu Phe
   1               5                   10                  15
   Ile Asn Val Pro Glu Trp Gly
               20
<210> 418
   <211> 23
   <212> PRT
   <213> Influenza A virus
<400> 418
   Lys Asp Ser Pro Ala Asn Asp Leu Gly Asn Pro Met Ser Asp Glu Phe
   1               5                   10                  15
   Leu Asn Val Pro Glu Trp Gly
               20
<210> 419
   <211> 23
   <212> PRT
   <213> Influenza A virus
<400> 419
   Lys Asp Ser Pro Ala Asn Gly Leu Gly Asn Pro Met Ser Asp Glu Phe
   1               5                   10                  15
   Ile Asn Val Pro Glu Trp Gly
               20
<210> 420
   <211> 23
   <212> PRT
   <213> Influenza A virus
<400> 420
   Lys Asp Ser Pro Ala Asn Gly Leu Gly Asn Pro Met Ser Asp Glu Phe
   1               5                   10                  15
   Leu Asn Val Pro Glu Trp Gly
               20
<210> 421
   <211> 23
   <212> PRT
   <213> Influenza A virus
<400> 421
   Lys Asp Ser Pro Val Asn Asp Leu Gly Asn Pro Met Ser Asp Glu Phe
   1               5                   10                  15
   Ile Asn Val Pro Glu Trp Gly
               20
<210> 422
   <211> 23
   <212> PRT
   <213> Influenza A virus
<400> 422
   Lys Asp Ser Pro Val Asn Asp Leu Gly Asn Pro Met Ser Asp Glu Phe
   1               5                   10                  15
   Leu Asn Val Pro Glu Trp Gly
               20
<210> 423
   <211> 23
   <212> PRT
   <213> Influenza A virus
<400> 423
   Lys Asp Ser Pro Val Asn Gly Leu Gly Asn Pro Met Ser Asp Glu Phe
   1               5                   10                  15
   Ile Asn Val Pro Glu Trp Gly
               20
<210> 424
   <211> 23
   <212> PRT
   <213> Influenza A virus
<400> 424
   Lys Asp Ser Pro Val Asn Gly Leu Gly Asn Pro Met Ser Asp Glu Phe
   1               5                   10                  15
   Leu Asn Val Pro Glu Trp Gly
               20
<210> 425
   <211> 23
   <212> PRT
   <213> Influenza A virus
<400> 425
   Pro Tyr Gln Gly Lys Ser Ser Phe Phe Arg Asn Val Val Trp Leu Ile
   1               5                   10                  15
   Lys Lys Asn Ser Ala Tyr Gly
               20
<210> 426
   <211> 23
   <212> PRT
   <213> Influenza A virus
<400> 426
   Pro Tyr Gln Gly Lys Ser Ser Phe Phe Arg Asn Val Val Trp Leu Ile
   1               5                   10                  15
   Lys Lys Asn Ser Thr Tyr Gly
               20
<210> 427
   <211> 23
   <212> PRT
   <213> Influenza A virus
<400> 427
   Pro Tyr Gln Gly Lys Ser Ser Phe Phe Arg Asn Val Val Trp Leu Ile
   1               5                   10                  15
   Lys Lys Asn Ser Val Tyr Gly
               20
<210> 428
   <211> 23
   <212> PRT
   <213> Influenza A virus
<400> 428
   Pro Tyr Asn Gly Lys Ser Ser Phe Phe Arg Asn Val Val Trp Leu Ile
   1               5                   10                  15
   Lys Lys Asn Ser Ala Tyr Gly
               20
<210> 429
   <211> 23
   <212> PRT
   <213> Influenza A virus
<400> 429
   Pro Tyr Asn Gly Lys Ser Ser Phe Phe Arg Asn Val Val Trp Leu Ile
   1 5 10 15
   Lys Lys Asn Ser Thr Tyr Gly
   20
<210> 430
   <211> 23
   <212> PRT
   <213> Influenza A virus
<400> 430
   Pro Tyr Asn Gly Lys Ser Ser Phe Phe Arg Asn Val Val Trp Leu Ile
   1 5 10 15
   Lys Lys Asn Ser Val Tyr Gly
   20
<210> 431
   <211> 23
   <212> PRT
   <213> Influenza A virus
<400> 431
   Pro Tyr Leu Gly Lys Ser Ser Phe Phe Arg Asn Val Val Trp Leu Ile
   1 5 10 15
   Lys Lys Asn Ser Ala Tyr Gly
   20
<210> 432
   <211> 23
   <212> PRT
   <213> Influenza A virus
<400> 432
   Pro Tyr Leu Gly Lys Ser Ser Phe Phe Arg Asn Val Val Trp Leu Ile
   1 5 10 15
   Lys Lys Asn Ser Thr Tyr Gly
   20
<210> 433
   <211> 23
   <212> PRT
   <213> Influenza A virus
<400> 433
   Pro Tyr Leu Gly Lys Ser Ser Phe Phe Arg Asn Val Val Trp Leu Ile
   1 5 10 15
   Lys Lys Asn Ser Val Tyr Gly
   20
<210> 434
   <211> 23
   <212> PRT
   <213> Influenza A virus
<400> 434
   Pro Tyr Gln Gly Lys Ser Ser Phe Phe Arg Asn Val Val Trp Leu Ile
   1 5 10 15
   Lys Lys Asn Asn Ala Tyr Gly
   20
<210> 435
   <211> 23
   <212> PRT
   <213> Influenza A virus
<400> 435
   Pro Tyr Gln Gly Lys Ser Ser Phe Phe Arg Asn Val Val Trp Leu Ile
   1 5 10 15
   Lys Lys Asn Asn Thr Tyr Gly
   20
<210> 436
   <211> 23
   <212> PRT
   <213> Influenza A virus
<400> 436
   Pro Tyr Gln Gly Lys Ser Ser Phe Phe Arg Asn Val Val Trp Leu Ile
   1 5 10 15
   Lys Lys Asn Asn Val Tyr Gly
   20
<210> 437
   <211> 23
   <212> PRT
   <213> Influenza A virus
<400> 437
   Pro Tyr Asn Gly Lys Ser Ser Phe Phe Arg Asn Val Val Trp Leu Ile
   1 5 10 15
   Lys Lys Asn Asn Ala Tyr Gly
   20
<210> 438
   <211> 23
   <212> PRT
   <213> Influenza A virus
<400> 438
   Pro Tyr Asn Gly Lys Ser Ser Phe Phe Arg Asn Val Val Trp Leu Ile
   1 5 10 15
   Lys Lys Asn Asn Thr Tyr Gly
   20
<210> 439
   <211> 23
   <212> PRT
   <213> Influenza A virus
<400> 439
   Pro Tyr Asn Gly Lys Ser Ser Phe Phe Arg Asn Val Val Trp Leu Ile
   1 5 10 15
   Lys Lys Asn Asn Val Tyr Gly
   20
<210> 440
   <211> 23
   <212> PRT
   <213> Influenza A virus
<400> 440
   Pro Tyr Leu Gly Lys Ser Ser Phe Phe Arg Asn Val Val Trp Leu Ile
   1 5 10 15
   Lys Lys Asn Asn Ala Tyr Gly
   20
<210> 441
   <211> 23
   <212> PRT
   <213> Influenza A virus
<400> 441
   Pro Tyr Leu Gly Lys Ser Ser Phe Phe Arg Asn Val Val Trp Leu Ile
   1 5 10 15
   Lys Lys Asn Asn Thr Tyr Gly
   20
<210> 442
   <211> 23
   <212> PRT
   <213> Influenza A virus
<400> 442
   Pro Tyr Leu Gly Lys Ser Ser Phe Phe Arg Asn Val Val Trp Leu Ile
   1 5 10 15
   Lys Lys Asn Asn Val Tyr Gly
   20
<210> 443
   <211> 23
   <212> PRT
   <213> Influenza A virus
<400> 443
   Pro Tyr Gln Gly Arg Ser Ser Phe Phe Arg Asn Val Val Trp Leu Ile
   1               5                   10                  15
   Lys Lys Asn Ser Ala Tyr Gly
               20
<210> 444
   <211> 23
   <212> PRT
   <213> Influenza A virus
<400> 444
   Pro Tyr Gln Gly Arg Ser Ser Phe Phe Arg Asn Val Val Trp Leu Ile
   1               5                   10                  15
   Lys Lys Asn Ser Thr Tyr Gly
               20
<210> 445
   <211> 23
   <212> PRT
   <213> Influenza A virus
<400> 445
   Pro Tyr Gln Gly Arg Ser Ser Phe Phe Arg Asn Val Val Trp Leu Ile
   1               5                   10                  15
   Lys Lys Asn Ser Val Tyr Gly
               20
<210> 446
   <211> 23
   <212> PRT
   <213> Influenza A virus
<400> 446
   Pro Tyr Asn Gly Arg Ser Ser Phe Phe Arg Asn Val Val Trp Leu Ile
   1               5                   10                  15
   Lys Lys Asn Ser Ala Tyr Gly
               20
<210> 447
   <211> 23
   <212> PRT
   <213> Influenza A virus
<400> 447
   Pro Tyr Asn Gly Arg Ser Ser Phe Phe Arg Asn Val Val Trp Leu Ile
   1               5                   10                  15
   Lys Lys Asn Ser Thr Tyr Gly
               20
<210> 448
   <211> 23
   <212> PRT
   <213> Influenza A virus
<400> 448
   Pro Tyr Asn Gly Arg Ser Ser Phe Phe Arg Asn Val Val Trp Leu Ile
   1               5                   10                  15
   Lys Lys Asn Ser Val Tyr Gly
               20
<210> 449
   <211> 23
   <212> PRT
   <213> Influenza A virus
<400> 449
   Pro Tyr Leu Gly Arg Ser Ser Phe Phe Arg Asn Val Val Trp Leu Ile
   1               5                   10                  15
   Lys Lys Asn Ser Ala Tyr Gly
               20
<210> 450
   <211> 23
   <212> PRT
   <213> Influenza A virus
<400> 450
   Pro Tyr Leu Gly Arg Ser Ser Phe Phe Arg Asn Val Val Trp Leu Ile
   1               5                   10                  15
   Lys Lys Asn Ser Thr Tyr Gly
   20
<210> 451
   <211> 23
   <212> PRT
   <213> Influenza A virus
<400> 451
   Pro Tyr Leu Gly Arg Ser Ser Phe Phe Arg Asn Val Val Trp Leu Ile
   1 5 10 15
   Lys Lys Asn Ser Val Tyr Gly
   20
<210> 452
   <211> 23
   <212> PRT
   <213> Influenza A virus
<400> 452
   Pro Tyr Gln Gly Arg Ser Ser Phe Phe Arg Asn Val Val Trp Leu Ile
   1 5 10 15
   Lys Lys Asn Asn Ala Tyr Gly
   20
<210> 453
   <211> 23
   <212> PRT
   <213> Influenza A virus
<400> 453
   Pro Tyr Gln Gly Arg Ser Ser Phe Phe Arg Asn Val Val Trp Leu Ile
   1 5 10 15
   Lys Lys Asn Asn Thr Tyr Gly
   20
<210> 454
   <211> 23
   <212> PRT
   <213> Influenza A virus
<400> 454
   Pro Tyr Gln Gly Arg Ser Ser Phe Phe Arg Asn Val Val Trp Leu Ile
   1 5 10 15
   Lys Lys Asn Asn Val Tyr Gly
   20
<210> 455
   <211> 23
   <212> PRT
   <213> Influenza A virus
<400> 455
   Pro Tyr Asn Gly Arg Ser Ser Phe Phe Arg Asn Val Val Trp Leu Ile
   1 5 10 15
   Lys Lys Asn Asn Ala Tyr Gly
   20
<210> 456
   <211> 23
   <212> PRT
   <213> Influenza A virus
<400> 456
   Pro Tyr Asn Gly Arg Ser Ser Phe Phe Arg Asn Val Val Trp Leu Ile
   1               5                   10                  15
   Lys Lys Asn Asn Thr Tyr Gly
               20
<210> 457
   <211> 23
   <212> PRT
   <213> Influenza A virus
<400> 457
   Pro Tyr Asn Gly Arg Ser Ser Phe Phe Arg Asn Val Val Trp Leu Ile
   1               5                   10                  15
   Lys Lys Asn Asn Val Tyr Gly
               20
<210> 458
   <211> 23
   <212> PRT
   <213> Influenza A virus
<400> 458
   Pro Tyr Leu Gly Arg Ser Ser Phe Phe Arg Asn Val Val Trp Leu Ile
   1               5                   10                  15
   Lys Lys Asn Asn Ala Tyr Gly
               20
<210> 459
   <211> 23
   <212> PRT
   <213> Influenza A virus
<400> 459
   Pro Tyr Leu Gly Arg Ser Ser Phe Phe Arg Asn Val Val Trp Leu Ile
   1               5                   10                  15
   Lys Lys Asn Asn Thr Tyr Gly
               20
<210> 460
   <211> 23
   <212> PRT
   <213> Influenza A virus
<400> 460
   Pro Tyr Leu Gly Arg Ser Ser Phe Phe Arg Asn Val Val Trp Leu Ile
   1 5 10 15
   Lys Lys Asn Asn Val Tyr Gly
   20
<210> 461
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 461
   Pro Tyr Gln Gly Lys Ser Ser Phe Phe Arg Asn Val Val Trp Leu Ile
   1 5 10 15
   Lys Lys Asn Ser Ala Tyr Gly
   20
<210> 462
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 462
   Pro Tyr Gln Gly Lys Ser Ser Phe Phe Arg Asn Val Val Trp Leu Ile
   1 5 10 15
   Lys Lys Asn Ser Thr Tyr Gly
   20
<210> 463
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 463
   Pro Tyr Gln Gly Lys Ser Ser Phe Phe Arg Asn Val Val Trp Leu Ile
   1 5 10 15
   Lys Lys Asn Ser Val Tyr Gly
   20
<210> 464
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 464
   Pro Tyr Asn Gly Lys Ser Ser Phe Phe Arg Asn Val Val Trp Leu Ile
   1 5 10 15
   Lys Lys Asn Ser Ala Tyr Gly
   20
<210> 465
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 465
   Pro Tyr Asn Gly Lys Ser Ser Phe Phe Arg Asn Val Val Trp Leu Ile
   1 5 10 15
   Lys Lys Asn Ser Thr Tyr Gly
   20
<210> 466
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 466
   Pro Tyr Asn Gly Lys Ser Ser Phe Phe Arg Asn Val Val Trp Leu Ile
   1 5 10 15
   Lys Lys Asn Ser Val Tyr Gly
   20
<210> 467
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 467
   Pro Tyr Leu Gly Lys Ser Ser Phe Phe Arg Asn Val Val Trp Leu Ile
   1 5 10 15
   Lys Lys Asn Ser Ala Tyr Gly
   20
<210> 468
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 468
   Pro Tyr Leu Gly Lys Ser Ser Phe Phe Arg Asn Val Val Trp Leu Ile
   1 5 10 15
   Lys Lys Asn Ser Thr Tyr Gly
   20
<210> 469
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 469
   Pro Tyr Leu Gly Lys Ser Ser Phe Phe Arg Asn Val Val Trp Leu Ile
   1               5                   10                  15
   Lys Lys Asn Ser Val Tyr Gly
               20
<210> 470
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 470
   Pro Tyr Gln Gly Lys Ser Ser Phe Phe Arg Asn Val Val Trp Leu Ile
   1               5                   10                  15
   Lys Lys Asn Asn Ala Tyr Gly
               20
<210> 471
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 471
   Pro Tyr Gln Gly Lys Ser Ser Phe Phe Arg Asn Val Val Trp Leu Ile
   1               5                   10                  15
   Lys Lys Asn Asn Thr Tyr Gly
               20
<210> 472
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 472
   Pro Tyr Gln Gly Lys Ser Ser Phe Phe Arg Asn Val Val Trp Leu Ile
   1 5 10 15
   Lys Lys Asn Asn Val Tyr Gly
   20
<210> 473
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 473
   Pro Tyr Asn Gly Lys Ser Ser Phe Phe Arg Asn Val Val Trp Leu Ile
   1 5 10 15
   Lys Lys Asn Asn Ala Tyr Gly
   20
<210> 474
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 474
   Pro Tyr Asn Gly Lys Ser Ser Phe Phe Arg Asn Val Val Trp Leu Ile
   1 5 10 15
   Lys Lys Asn Asn Thr Tyr Gly
   20
<210> 475
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 475
   Pro Tyr Asn Gly Lys Ser Ser Phe Phe Arg Asn Val Val Trp Leu Ile
   1               5                   10                  15
   Lys Lys Asn Asn Val Tyr Gly
               20
<210> 476
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 476
   Pro Tyr Leu Gly Lys Ser Ser Phe Phe Arg Asn Val Val Trp Leu Ile
   1               5                   10                  15
   Lys Lys Asn Asn Ala Tyr Gly
               20
<210> 477
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 477
   Pro Tyr Leu Gly Lys Ser Ser Phe Phe Arg Asn Val Val Trp Leu Ile
   1               5                   10                  15
   Lys Lys Asn Asn Thr Tyr Gly
               20
<210> 478
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 478
   Pro Tyr Leu Gly Lys Ser Ser Phe Phe Arg Asn Val Val Trp Leu Ile
   1               5                   10                  15
   Lys Lys Asn Asn Val Tyr Gly
               20
<210> 479
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 479
   Pro Tyr Gln Gly Arg Ser Ser Phe Phe Arg Asn Val Val Trp Leu Ile
   1               5                   10                  15
   Lys Lys Asn Ser Ala Tyr Gly
               20
<210> 480
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 480
   Pro Tyr Gln Gly Arg Ser Ser Phe Phe Arg Asn Val Val Trp Leu Ile
   1               5                   10                  15
   Lys Lys Asn Ser Thr Tyr Gly
               20
<210> 481
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 481
   Pro Tyr Gln Gly Arg Ser Ser Phe Phe Arg Asn Val Val Trp Leu Ile
   1 5 10 15
   Lys Lys Asn Ser Val Tyr Gly
   20
<210> 482
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 482
   Pro Tyr Asn Gly Arg Ser Ser Phe Phe Arg Asn Val Val Trp Leu Ile
   1 5 10 15
   Lys Lys Asn Ser Ala Tyr Gly
   20
<210> 483
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 483
   Pro Tyr Asn Gly Arg Ser Ser Phe Phe Arg Asn Val Val Trp Leu Ile
   1 5 10 15
   Lys Lys Asn Ser Thr Tyr Gly
   20
<210> 484
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 484
   Pro Tyr Asn Gly Arg Ser Ser Phe Phe Arg Asn Val Val Trp Leu Ile
   1 5 10 15
   Lys Lys Asn Ser Val Tyr Gly
   20
<210> 485
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 485
   Pro Tyr Leu Gly Arg Ser Ser Phe Phe Arg Asn Val Val Trp Leu Ile
   1 5 10 15
   Lys Lys Asn Ser Ala Tyr Gly
   20
<210> 486
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 486
   Pro Tyr Leu Gly Arg Ser Ser Phe Phe Arg Asn Val Val Trp Leu Ile
   1 5 10 15
   Lys Lys Asn Ser Thr Tyr Gly
   20
<210> 487
   <211> 23
   <212> PRT
   <213> Influenza A virus

<220>
   <221> MISC_FEATURE
   <222> (1)..(1)
   <223> residue lipidated, for example with di-palmitoyl-Lys-Ser-Ser-
<400> 487
   Pro Tyr Leu Gly Arg Ser Ser Phe Phe Arg Asn Val Val Trp Leu Ile
   1 5 10 15
   Lys Lys Asn Ser Val Tyr Gly
   20
<210> 488
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 488
   Pro Tyr Gln Gly Arg Ser Ser Phe Phe Arg Asn Val Val Trp Leu Ile
   1 5 10 15
   Lys Lys Asn Asn Ala Tyr Gly
   20
<210> 489
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 489
   Pro Tyr Gln Gly Arg Ser Ser Phe Phe Arg Asn Val Val Trp Leu Ile
   1 5 10 15
   Lys Lys Asn Asn Thr Tyr Gly
   20
<210> 490
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 490
   Pro Tyr Gln Gly Arg Ser Ser Phe Phe Arg Asn Val Val Trp Leu Ile
   1 5 10 15
   Lys Lys Asn Asn Val Tyr Gly
   20
<210> 491
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 491
   Pro Tyr Asn Gly Arg Ser Ser Phe Phe Arg Asn Val Val Trp Leu Ile
   1 5 10 15
   Lys Lys Asn Asn Ala Tyr Gly
   20
<210> 492
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 492
   Pro Tyr Asn Gly Arg Ser Ser Phe Phe Arg Asn Val Val Trp Leu Ile
   1 5 10 15
   Lys Lys Asn Asn Thr Tyr Gly
   20
<210> 493
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 493
   Pro Tyr Asn Gly Arg Ser Ser Phe Phe Arg Asn Val Val Trp Leu Ile
   1               5                   10                  15
   Lys Lys Asn Asn Val Tyr Gly
               20
<210> 494
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 494
   Pro Tyr Leu Gly Arg Ser Ser Phe Phe Arg Asn Val Val Trp Leu Ile
   1               5                   10                  15
   Lys Lys Asn Asn Ala Tyr Gly
               20
<210> 495
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 495
   Pro Tyr Leu Gly Arg Ser Ser Phe Phe Arg Asn Val Val Trp Leu Ile
   1               5                   10                  15
   Lys Lys Asn Asn Thr Tyr Gly
               20
<210> 496
   <211> 23
   <212> PRT
   <213> Influenza A virus
<220>
   <221> MISC_FEATURE
   <222> (1)..(1)
   <223> residue lipidated, for example with di-palmitoyl-Lys-Ser-Ser-
<400> 496
   Pro Tyr Leu Gly Arg Ser Ser Phe Phe Arg Asn Val Val Trp Leu Ile
   1               5                   10                  15
   Lys Lys Asn Asn Val Tyr Gly
               20

## Description

### FIELD OF THE INVENT10N

The present invention relates generally to an anti-viral formulation, and in particular relates to a peptide-based influenza vaccine formulation, more particularly to an avian influenza peptide-based vaccine formulation.

### BACKGROUND OF THE INVENTION

Avian influenza is an infectious disease of birds caused by type A strains of the influenza virus. The disease, which was first identified in Italy more than 100 years ago, occurs worldwide. Sixteen subtypes of influenza virus are known to infect birds, thus providing an extensive reservoir of influenza viruses potentially circulating in bird populations. To date, all known outbreaks of the highly pathogenic form have been caused by influenza A viruses of subtypes H5 and H7.

Of the 16 avian influenza virus subtypes, H5N1 is of particular concern for several reasons. H5N1 mutates rapidly and has a documented propensity to acquire genes from viruses, thereby facilitating infection of other animal species. Indeed, its ability to cause severe disease in humans has now been documented. Laboratory studies have demonstrated that isolates from this virus have a high pathogenicity and can cause mortality in humans.

Two other avian influenza viruses have recently been found to cause illness in humans: H7N7 and H9N2.

All type A influenza viruses are genetically labile and well adapted to elude host defences. Influenza viruses lack mechanisms for the "proofreading" and repair of errors that occur during replication. As a result of these uncorrected errors, the genetic composition of the viruses changes as they replicate in humans and animals, and new antigenic variants emerge. These constant, permanent and usually small changes in the antigenic composition of influenza A viruses are known as antigenic "drift".

Influenza viruses are typed as A or B on the basis of relatively stable intracellular nucleoproteins and envelope associated matrix proteins. Virus subtypes are based on two proteins in the viral envelope, hemagglutinin (HA) and neuraminidase (NA), which undergo constant antigenic change. 16 distinct subtypes of HA and 9 subtypes of NA are recognized for influenza A viruses. The sudden appearance of a new subtype (antigenic shift) has caused three major pandemics in the past century: 1918 (Spanish Flu, H1N1), 1957 (Asian Flu, H2N2) and 1968 (Hong Kong Flu, H3N2).

Influenza viruses have a second characteristic of great public health concern: influenza A viruses can swap or "re-assort" genetic materials between subtypes of any species resulting in novel subtypes. This reassortment process, known as antigenic "shift," has resulted in worldwide pandemics in humans.

Influenza pandemics have occurred, on average, three to four times each century when new virus subtypes have emerged that are readily transmitted from person to person. In the 20th century, the great influenza pandemic of 1918 -1919, which caused an estimated 40 to 50 million deaths worldwide, was followed by pandemics in 1957-1958 and 1968 -1969. Experts surmise that another influenza pandemic is inevitable and possibly imminent. Given the unpredictable behaviour of influenza viruses, neither the timing nor the severity of the next pandemic can be predicted with any certainty.

Seven variable B-cell epitopes, and one variable T-cell epitope collectively represent the antigenic drift sites found on the hemagglutinin HA1 protein of Influenza A (subtype H5). Each of the B-cell variable epitopes represents a conformational epitope, and four of them are comprised of two discontinuous stretches of amino acids. There are two extended antigenic sites on the HA1 proteins, and each of them is represented by two distinct peptide sequences. The nonadjacent segments (stretches of amino acids) that are artificially joined together to represent the discontinuous epitopes are selected using the three-dimensional structure of A/duck/Singapore/3/97 hemagglutinin (PDB ID code: 1JSM). Use of crystallographic data aids in design of linear peptides that can mimic the native conformational epitopes of proteins. The T-cell eptiope is represented by a linear peptide sequence which may also be lipidated.

To date, no effective peptide-based vaccine against avian influenza is commercially available.

Current antiviral therapies may be clinically effective against influenza A virus strains in otherwise healthy adults and children; however, these therapies have limitations. Some of these drugs are expensive and supplies are limited. The vaccine composition must also change each year to account for changes in the virus circulating in the population due to antigenic drift. At least four months of development time is required to produce a new effective vaccine in significant quantities.

Processes for preparation of an immunogenic peptide mixture are described by Torres in U.S. Patent No. 7,118,874, and in PCT application PCT/CA06/000891. According to one of these processes, the variability of immunogenic epitope sequences of a pathogen are evaluated. A peptide mixture is synthesized comprising a plurality of peptides representative of the frequency with which different amino acids are found at variable residues of selected epitopes.

D1 (Katz et al Biomed and Pharmacother 2000 vol 54 pages 178-87) discloses pathogenesis of and immunity to avian influenza A h5 viruses. The BALB/c mouse was used to better understand the pathogenesis of and immunity to the H5N1 viruses in a mammalian model.

D2 (US2004/0223976A1) discloses an influenza virus vaccine. Vaccines against disease caused by infection with influenza virus and methods of vaccination are disclosed. The vaccines comprise peptides derived from the M2 and/or HA proteins of influenza virus conjugated to a carrier protein.

D3 (WO03/066090A1) discloses immunogenic formulations of variable peptidic epitopes and process for preparation thereof. A process is disclosed for preparation of an immunogenic peptide mixture in a single synthesis.

D4 (Kaverin et al J. General Virol. 2002 vol 83 pages 2497-2505) discloses structure of antigenic sites on the haemagglutinin molecule of H5 avian influenza virus and phenotypic variation of escape mutants.

Thus, there is a need to develop a vaccine formulation effective against multiple subtypes and multiple variants of avian influenza.

### SUMMARY OF THE INVENTION

It is an object of the present invention to obviate or mitigate at least one disadvantage of previous influenza vaccine formulations.

In a first aspect, the present invention provides a peptide-based anti-influenza formulation consisting of a mixture of peptides, the sequences of the peptides in the mixture consisting of SEQ ID. NOs: 1 to 40.
In another aspect, the invention relates to a vaccine comprising the formulation as described above together with a pharmaceutically-acceptable diluent or carrier.
Suitably the vaccine further comprises an adjuvant. Suitably the adjuvant is alum.
In another aspect, the invention relates to use of the formulation as described above for the preparation of a vaccine for preventing or treating influenza in an animal in need thereof. Suitably the influenza is avian influenza.
In another aspect, the invention relates to a vaccine as described above for use in preventing or treating influenza. Suitably the influenza is avian influenza.

The anti-viral formulation can be an anti-influenza formulation. More particularly, the anti-influenza formulation can be an avian anti-influenza formulation.

In a further aspect of the present invention, there is provided a use of the formulation as described above, for the preparation of a vaccine. The vaccine can be used for preventing or treating influenza in an animal in need thereof. In one exemplary embodiment, the influenza is avian influenza. The present invention further relates to a method for inducing an immune response in humans or animals and conferring protection against avian influenza, or novel subtypes of influenza derived from avian influenza, which comprises administering to humans or other animals a peptide-based vaccine as described herein.

There is described a method for preparing an anti-viral formulation, such as the anti-viral formulation as described herein. There is described a method for preparing a peptide from SEQ ID NOs: 1 to 212 comprising the steps of determining a linear sequence representative of primary sequences of discontinuous epitopes of an avian influenza viral protein, wherein the epitopes are in proximity to each other when the protein is in a folded conformation; and synthesizing a peptide representative of the linear sequence. There is described a method for preparing a peptide mixture comprising any two peptide sequences from SEQ ID NOs: 1 to 212 comprising the steps of: determining a linear sequence representative of primary sequences of discontinuous epitopes of an avian influenza viral protein, the epitopes being in proximity to each other when the protein is in a folded conformation; said discontinuous epitopes comprising variable residues, and synthesizing a peptide mixture including at least two different amino acids at a variable residue.

There is described an anti-influenza vaccine comprising a mixture of peptides containing at least one hemagglutinin (HA) antigen of influenza virus. Hemagglutinin (HA) is a potent immunogen, and viral neutralizing antibodies are directed against the variable regions of HA. The isolated peptide mixture represents variants of multiple variable regions of hemagglutinin. Thus, there is described an anti-viral formulation comprising a mixture of isolated peptides, said mixture being formulated on the basis of the variable region of the avian influenza virus HA protein and said isolated peptide mixture representing variants of a variable region of the HA or HA1 protein, wherein each of said variable regions comprising a plurality of variable amino acid residues, at least one of which is represented by two or more amino acids. For example the plurality of variable amino acid residues in the anti-viral formulation comprises three or more residues. One or more of said Avian influenza proteins can be an HA or HA1.

Other aspects and features of the present invention will become apparent to those ordinarily skilled in the art upon review of the following description in conjunction with the accompanying Figures.

### BRIEF DESCRIPTION OF THE DRAWINGS

Embodiments of the present invention will now be described, by way of example only, with reference to the attached Figures, wherein:
Fig. 1 shows an analytical HPLC chromatogram of crude INFA-H5-1-V1 peptide sequences (corresponding to SEQ ID NOs: 249 to 272).
Fig. 2 shows an analytical HPLC chromatogram of crude INFA-H5-1-V2 peptide sequences (corresponding SEQ ID NOs: 273 to 288).
Fig. 3 shows an analytical HPLC chromatogram of crude INFA-H5-1-V3 peptide sequences (corresponding to SEQ ID NOs: 289 to 312).
Fig. 4 shows an analytical HPLC chromatogram of crude INFA-H5-1-V4 peptide sequences (corresponding to SEQ ID NOs: 313 to 336).
Fig. 5 shows an analytical HPLC chromatogram of crude INFA-H5-1-V5 peptide sequences (corresponding to SEQ ID NOs: 337 to 368).
Fig. 6 shows an analytical HPLC chromatogram of crude INFA-HS-1-V6 peptide sequences (corresponding to SEQ ID NOs: 369 to 392).
Fig. 7 shows an analytical HPLC chromatogram of crude INFA-H5-1-V7 peptide sequences (corresponding to SEQ ID NOs: 393 to 424).
Fig. 8 shows an analytical HPLC chromatogram of crude INFA-H5-1-V8 peptide sequences (corresponding to SEQ ID NOs: 425 to 460).
Fig. 9 shows a MALDI-TOF spectrum of crude INFA-H5-1-V1 peptide sequences (corresponding to SEQ ID NOs: 249 to 272).
Fig. 10 shows a MALDI-TOF spectrum of crude INFA-H5-1-V2 peptide sequences (corresponding to SEQ ID NOs: 273 to 288).
Fig. 11 shows a MALDI-TOF spectrum of crude INFA-H5-1-V3 peptide sequences (corresponding to SEQ ID NOs: 289 to 312).
Fig. 12 shows a MALDI-TOF spectrum of crude INFA-H5-1-V4 peptide sequences (corresponding to SEQ ID NOs: 313 to 336).
Fig. 13 shows a MALDI-TOF spectrum of crude INFA-H5-1-V5 peptide sequences (corresponding to SEQ ID NOs: 337 to 368).
Fig. 14 shows a MALDI-TOF spectrum of crude INFA-H5-1-V6 peptide sequences (corresponding to SEQ ID NOs: 369 to 392).
Fig. 15 shows a MALDI-TOF spectrum of crude INFA-H5-1-V7 peptide sequences (corresponding to SEQ ID NOs: 393 to 424).
Fig. 16 shows a MALDI-TOF spectrum of crude INFA-H5-1-V8 peptide sequences (corresponding to SEQ ID NOs: 425 to 460).
Fig. 17 shows a MALDI-TOF spectrum of crude lipidated INFA-H5-1-V8 peptide sequences (corresponding to SEQ ID NOs: 461 to 496).
Fig. 18(a)-(h) shows the different variosite peptide sequences of the present invention, with variable residues beneath the consensus sequence. Fig. 18(a) is INFA-H5-1-V1; Fig. 18(b) is INFA-H5-1-V2; Fig. 18(c) is INFA-H5-1-V3; Fig. 18(d) is INFA-H5-1-V4; Fig. 18(e) is INFA-H5-1-V5; Fig. 18(f) is INFA-H5-1-V6; Fig. 18(g) is INFA-H5-1-V7; Fig. 18(h) is INFA-H5-1-V8.
Fig. 18(i) shows different lipidated variosite peptide sequences based on the consensus sequence in Fig. 18(h).
Fig. 19 illustrates induction of humoral immunity by a vaccine of the present invention after immunization. Blue bar (top bar) = AviFlu vaccine INFA-02L + alum; Red bar (2^{nd} bar from top) = AviFlu vaccine INFA-02L without adjuvant; Purple bar (middle bar) = AviFlu vaccine INFA-02P + montanide; Green bar (2^{nd} bar from bottom) = AviFlu vaccine INFA-02P + alum; Black bar (bottom bar) = control.
Fig. 20 illustrates a survival plot of vaccinated mice against challenge with H5N1. Legend from top: Black = control; Green = INFA-02P + alum; Purple = INFA-02P + montanide; Red = INFA-02L without adjuvant; Blue = INFA-02L + alum.
Fig. 21 shows induction of humoral immunity by INFA-01P (INFA-HA-1-(V1-V2)) versus INFA-02P (INFA-HA-1-(V1-V8)) after vaccination in mice as measured by HAI titres. Blue bar (bottom bar) = INFA-01 P + montanide; Purple bar (middle bar) = INFA-02P + montanide; Black bar (top bar) = control.
Fig. 22 shows a survival plot of mice, vaccinated by INFA-01 P (INFA-HA-1-(V1-V2)), against challenge with H5N1. Legend from top: Black = control; Blue - INFA-01P + montanide.

### DETAILED DESCRIPTION

Generally described is an anti-influenza formulation, and, more specifically, a vaccine for Influenza A, including avian subtypes.

The present invention provides a peptide-based anti-influenza formulation consisting of a mixture of peptides, the sequences of the peptides in the mixture consisting of SEQ ID. NOs: 1 to 40.

The vaccine can further comprise an adjuvant. In one example, the adjuvant is alum.

The anti-viral formulation can be an anti-influenza formulation. More particularly, the anti-influenza formulation can be an avian anti-influenza formulation.

In a further aspect of the present invention, there is provided a use of the formulation for the preparation of a vaccine. The vaccine can be used for preventing or treating influenza in an animal in need thereof. In one exemplary embodiment, the influenza is avian influenza. The present invention further relates to a method for inducing an immune response in humans or animals and conferring protection against avian influenza, or novel subtypes of influenza derived from avian influenza, which comprises administering to humans or other animals a peptide-based vaccine as described herein.

In a further aspect of the present invention, there is provided a method for preparing an anti-viral formulation, such as the anti-viral formulation as described herein. There is described a method for preparing a peptide from SEQ ID NOs: 1 to 212 comprising the steps of determining a linear sequence representative of primary sequences of discontinuous epitopes of an avian influenza viral protein, wherein the epitopes are in proximity to each other when the protein is in a folded conformation; and synthesizing a peptide representative of the linear sequence. There is described a method for preparing a peptide mixture comprising any two peptide sequences from SEQ ID NOs: 1 to 212 comprising the steps of: determining a linear sequence representative of primary sequences of discontinuous epitopes of an avian influenza viral protein, the epitopes being in proximity to each other when the protein is in a folded conformation; said discontinuous epitopes comprising variable residues, and synthesizing a peptide mixture including at least two different amino acids at a variable residue.

Generally described are an anti-influenza vaccine comprising a mixture of peptides containing at least one hemagglutinin (HA) antigen of influenza virus. Hemagglutinin (HA) is a potent immunogen, and viral neutralizing antibodies are directed against the variable regions of HA. The isolated peptide mixture represents variants of multiple variable regions of hemagglutinin. Thus, described is an anti-viral formulation comprising a mixture of isolated peptides, said mixture being formulated on the basis of the variable region of the avian influenza virus HA protein and said isolated peptide mixture representing variants of a variable region of the HA or HA1 protein, wherein each of said variable regions comprising a plurality of variable amino acid residues, at least one of which is represented by two or more amino acids. In one example, the plurality of variable amino acid residues in the anti-viral formulation comprises three or more residues. One or more of said Avian influenza proteins can be an HA or HA1.

The vaccine may be formulated with or without representing variation at specific residues for each peptide. When variation is not represented, the peptide formed may be referred to herein as a Discotope^{™} construct. A discotope construct is a linear sequence synthetic construct that approximates the position of primary sequence sections that compose discontinuous epitopes. The individual sections are constructed in sequence to elicit immune responses that recognize the discontinuous epitopes found in the original intact protein.

Discontinuous epitopes are composed of two or more segments of the primary sequence of a protein that when properly folded come together and are bound by specific antibodies. They are not recognized by antibodies when the secondary structure is lost and therefore have not been represented by a continuous linear peptide.

When variation is present at particular residues that are known to have different amino acids represented according to different sequences for that particular pathogen, the formulation comprises a number of peptides, which may be collectively referred to herein as a Discosite^{™} construct.

### Design of eptiopes

Hemagglutinin is the major surface glycoprotein of influenza virus and a potent immunogen against which viral neutralizing antibodies are directed. We have designed eight peptides that mimic discontinuous B- and T-cell epitopes on antigenic sites of HA. The sequences of these peptides are determined based on analysis of the crystal structure of influenza hemagglutinin (HA) protein to determine peptide epitopes. Hemagglutinin is the major surface glycoprotein of influenza virus and a potent immunogen against which viral neutralizing antibodies are directed. The linear peptide epitopes in the cocktail mimic discontinuous epitopes on the HA protein surface. Using bioinformatics software that analyzes the antigenic variation of HA proteins from thousands of human influenza isolates, degenerative peptide cocktails based on these epitopes can be prepared which represent the antigenic variation of HA within these epitopes. Thus, the influenza vaccine formulations of the present invention comprise a cocktail of peptides that represent major epitopes of the HA protein.

HA is the major envelope glycoprotein of influenza virus, and mediates the penetration of virus into host cells. The native HA is formed by the association of three HA monomers which, as a precondition of virus infectivity, are cleaved enzymatically into the amino-terminal HA1 and carboxy-terminal HA2. Based on the three dimensional structure of HA1, antigenic sites have been mapped by determining the amino acid changes of antigenic variants. The antigenic variations were mostly seen surrounding the receptor binding region of HA. including residues around the antibody inaccessible receptor binding pockets. Monoclonal antibodies to these antigenic sites neutralize influenza virus infectivity when the exact sequences are present. Both T and B cell epitopes are found on these sites.

All amino acids changes documented in virus escape mutants, selected by MAB or other methods, were analyzed. The proteins were aligned, and position of those amino acids was mapped onto 3-D structure of hemagglutinin H5. The location of the epitope was roughly predicted in hemagglutinin H5 protein as the area surrounds amino acids that undergo the immune pressure. Antigenic sites were then redefined using the three-dimensional structure of A/duck/Singapore/3/97 hemagglutinin (PDB ID code: 1JSM) in a sense that antigenic determinants must be freely accessible for B-cell antibodies, and that different segments of same epitope must be in close proximity to each other (for example, within 20 A).

The occurrence of amino acids at variable sites within constructed epitopes was assessed by analyzing hemagglutinin HA1 strains of Influenza A (subtype H5) virus, available in the Los Alamos Data Base as of June 28, 2005. Either 460 from all hosts or only 38 human hemagglutinin HA1 strains were used for analysis. A variable residue was defined as a position in which the occurrence of the most frequent amino acid at that position is less than 85% among all viral sequences examined.

A plurality of variable amino acid residues may comprise three or more residues, with two or more different amino acids at each variable position.

Part or all of the peptides comprising an influenza vaccine may be lipidated.

Discontinuous epitopes are composed of two or more segments of the primary sequence of a protein that exist in close proximity when in a native, three-dimensional conformation. They are not recognized by antibodies when the secondary or tertiary structure is lost; thus, linear peptides cannot traditionally be used to represent discontinuous epitopes. Crystallographic data from influenza hemagglutinin was used to design linear sequences that represent at least five conformational epitopes.

From each variable epitope, the peptide length is selected, and within the peptide, a plurality of variable residues is selected. Each variable residue has at least two optional amino acids, found naturally occurring in sequenced versions of the virus. In this way, a high degree of variability is represented. For example, three or four variable residues may be represented in the mixture of peptides, each having two or more different amino acids represented in the sequenced database records for influenza variants. If two variable residues occur in a variable region, then 2² different peptides would be used in the mixture representing that particular region. If three or four variable residues are indicated in a hypervariable region, the number of peptides in the resulting mixture would be 2³ and 2⁴, respectively. Generally, if variable regions consist of A, B, C, and so on variable sites, with a, b, c, and so on different amino acids at respective site, the total number of peptides would be A^{a}xB^{b}xC^{c} and so on.

Once the proteins, variable epitopes, peptide lengths, and variable residues are selected, the synthesis of the peptide mixtures occurs, according to any acceptable method of peptide synthesis.

Peptide mixtures are synthesized with each different peptide sequence represented in roughly equimolar quantities. However, there is no requirement to provide equimolar quantities of the individual peptides.

Lipidation of peptides may be conducted by any conventional or acceptable route, as would be known to those of skill in the art. Peptides need not be lipidated, but it may be advantageous for certain peptides to be lipidated with any acceptable lipid, such as palmitic acid, so as to allow a peptide to pass through a cell membrane. Peptides incorporating lipid may benefit from placement of a KSS motif at the C-terminal. The peptides incorporating lipid may contain 1 or more lipid moieties, for example, two lipid moieties per peptide. Immunization with lipidated peptides may result in an enhanced cytotoxic T lymphocyte (CTL) response.

Peptides described (i.e., corresponding to SEQ ID NOs. 1-212 and SEQ ID NOs. 249-460) form 8 groups derived from H5 antigenic sites on hemagglutinin. These groups are identified as INFA-H5-1-V1, INFA-H5-1-V2, INFA-H5-1-V3, INFA-H5-1-V4, INFA-H5-1-V5, INFA-H5-1-V6, INFA-H5-1-V7 and INFA-H5-1-V8. The groups contain the following sequences:
Groups INFA-H5-1-V1 (SEQ ID NOs 1-24 and SEQ ID NOs. 249-272), INFA-H5-1-V3 (SEQ ID NOs 41-64 and SEQ ID NOs. 289-312), INFA-H5-1-V4 (SEQ ID NOs 65-88 and SEQ ID NOs. 313-336) and INFA-H5-1-V6 (SEQ ID NOs 121-144 and SEQ ID NOs. 369-392) consist of 24 peptide variants.
Groups INFA-H5-1-V5 (SEQ ID NOs 89-120 and SEQ ID NOs. 337-368) and INFA-H5-1-V7 (SEQ ID NOs 145-176 and SEQ ID NOs. 393-424) consist of 32 peptide variants.
Group INFA-H5-1-V2 (SEQ ID NOs 25-40 and SEQ ID NOs. 273-288) consists of 16 peptide variants.
Group INFA-H5-1-V8 (SEQ ID NOs 177-212 and SEQ ID NOs. 425-460) consists of 36 peptide variants.

During typical preparation of the peptide sequences, an additional residue (such as a glycine residue) may be added at an end of sequence. Sequences corresponding to peptides having an additional glycine residue are shown in SEQ ID NOs: 249 to 496. The additional glycine residue has no material effect on the function of the peptide, and the presence of the glycine residue is merely a product of peptide synthesis which would be well understood to the person of ordinary skill in the art. In addition, therefore, peptides normally synthesized in this manner would represent typical examples of "analogues" (as described below) of peptides described.

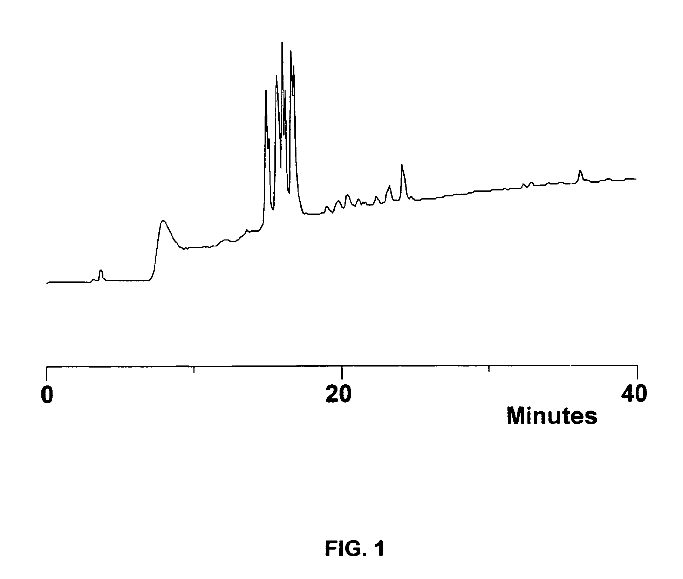

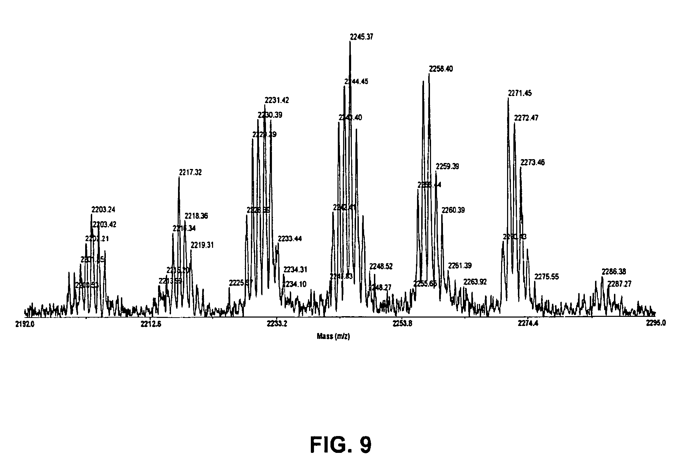

**Figs. 1** **and** **9** are related to peptide group INFA-H5-1-V1. Fig. 1 shows an analytical HPLC chromatogram of crude INFA-H5-1-V1 peptides. Fig. 9 shows a MALDI-TOF spectrum of crude INFA-H5-1-V1 peptides.

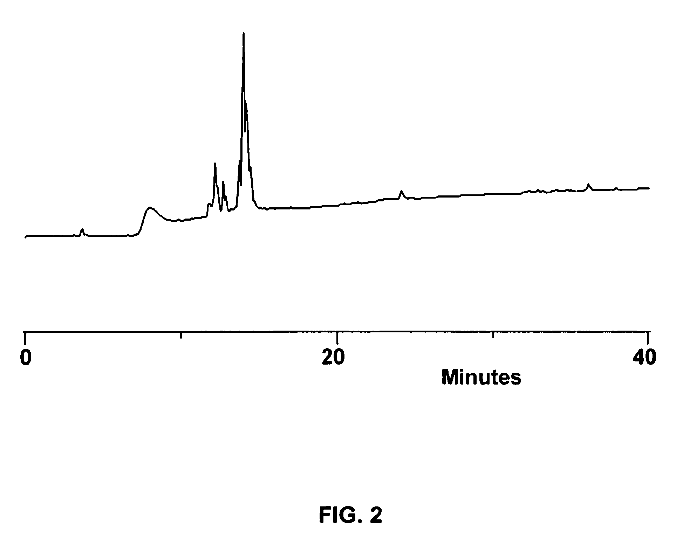

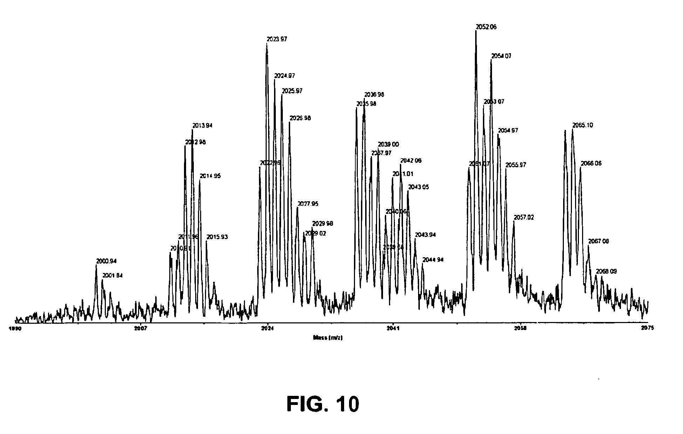

**Figs. 2** **and** **10** are related to peptide group INFA-H5-1-V2. Fig. 2 shows an analytical HPLC chromatogram of crude INFA-H5-1-V2 peptides. Fig. 10 shows a MALDI-TOF spectrum of crude INFA-H5-1-V2 peptides.

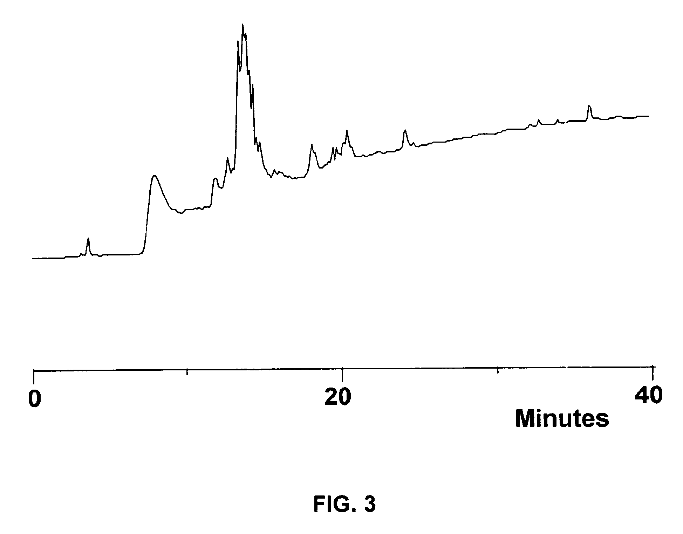

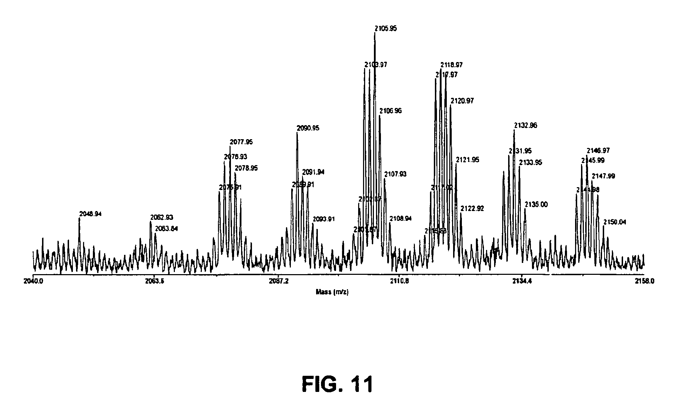

**Figs. 3** **and** **11** are related to peptide group INFA-H5-1-V3. Fig. 3 shows an analytical HPLC chromatogram of crude INFA-H5-1-V3 peptides. Fig. 11 shows a MALDI-TOF spectrum of crude INFA-H5-1-V3 peptides.

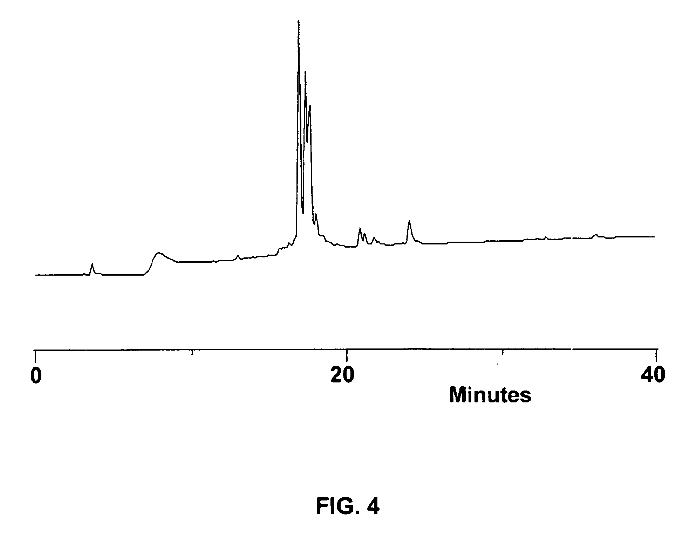

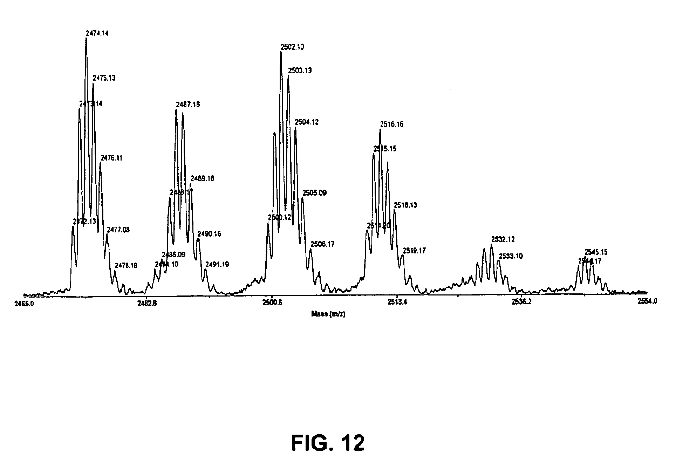

**Figs. 4** **and** **12** are related to peptide group INFA-H5-1-V4. Fig. 4 shows an analytical HPLC chromatogram of crude INFA-H5-1-V4 peptides. Fig. 12 shows a MALDI-TOF spectrum of crude INFA-H5-1-V4 peptides.

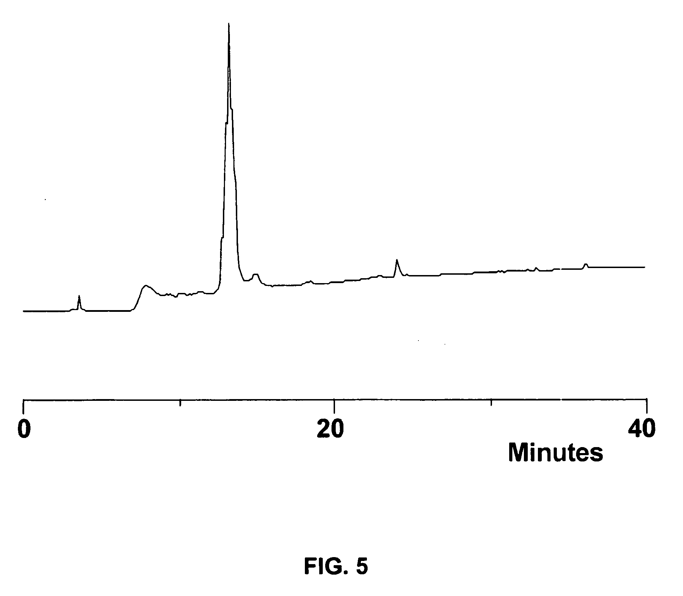

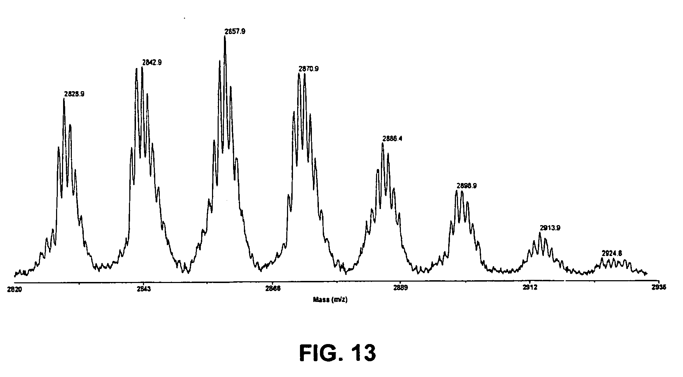

**Figs. 5** **and** **13** are related to peptide group INFA-H5-1-V5. Fig. 5 shows an analytical HPLC chromatogram of crude INFA-H5-1-V5 peptide. Fig. 13 shows a MALDI-TOF spectrum of crude INFA-H5-1-V5 peptides.

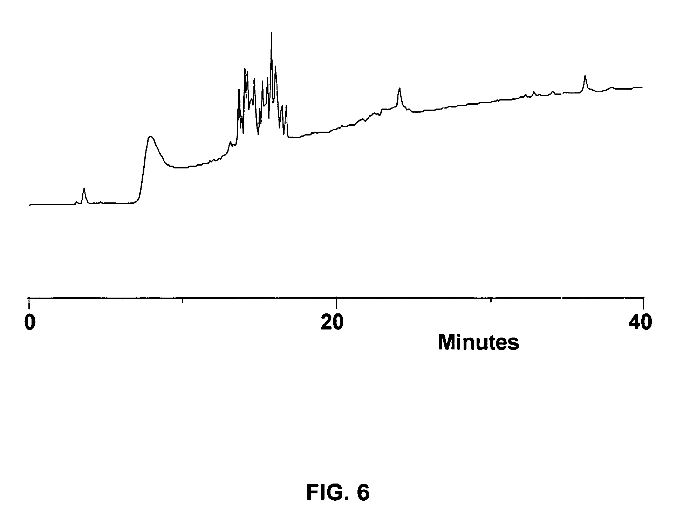

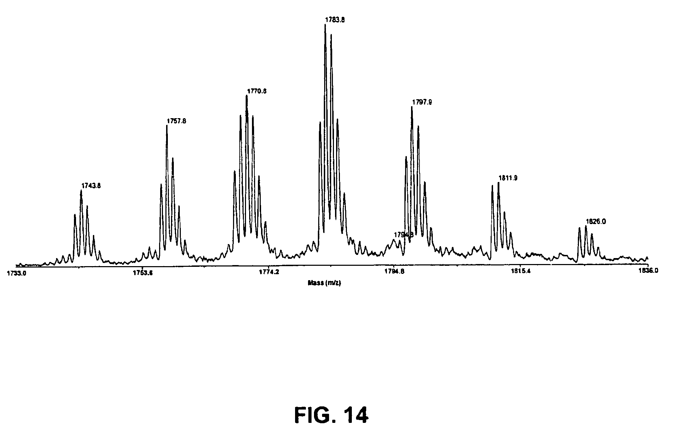

**Figs. 6** **and** **14** are related to peptide group INFA-H5-1-V6. Fig. 6 shows an analytical HPLC chromatogram of crude INFA-H5-1-V6 peptides. Fig. 14 shows a MALDI-TOF spectrum of crude INFA-H5-1-V6 peptides.

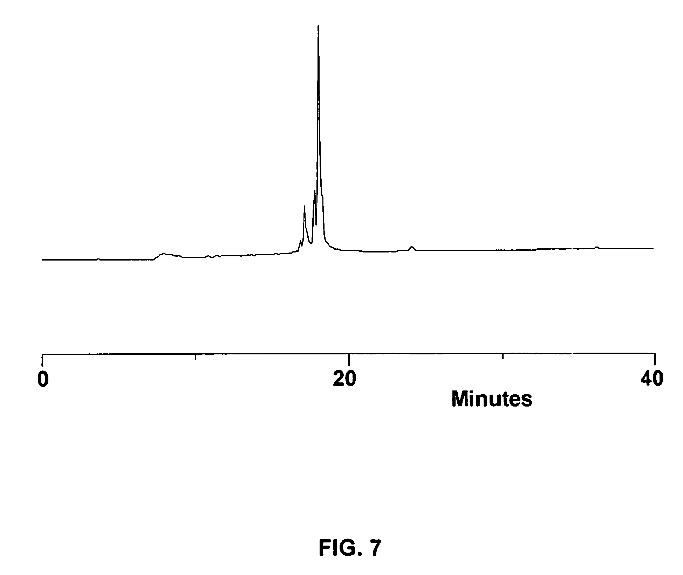

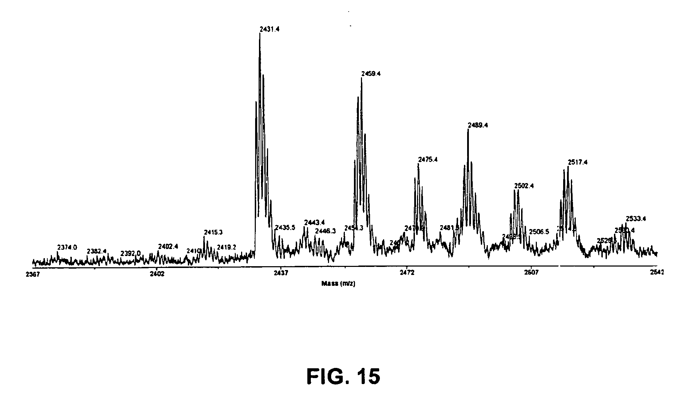

**Figs. 7** **and** **15** are related to peptide group INFA-H5-1-V7. Fig. 7 shows an analytical HPLC chromatogram of crude INFA-H5-1-V7 peptides. Fig. 15 shows a MALDI-TOF spectrum of crude INFA-H5-1-V7 peptides.

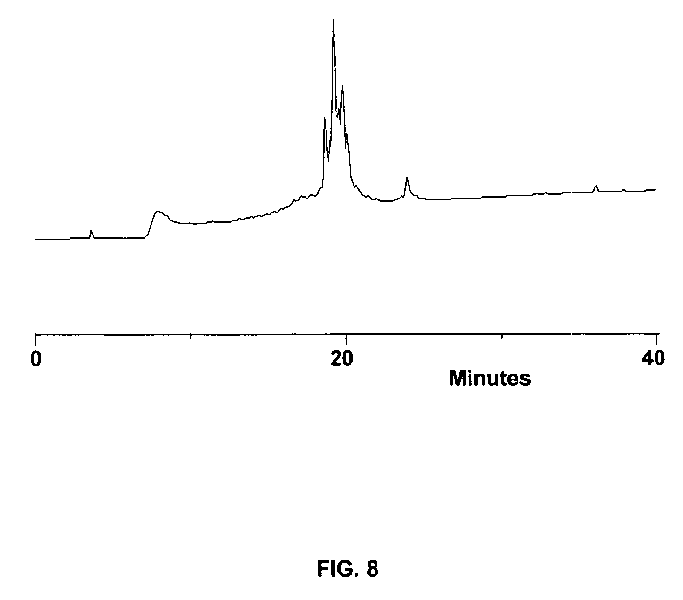

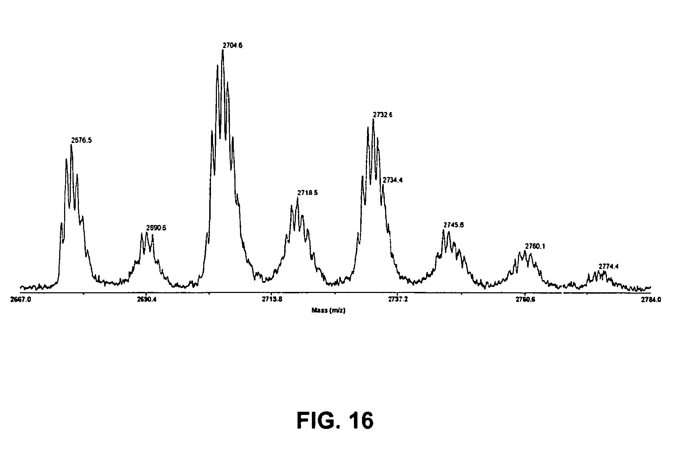

**Figs. 8** **and** **16** are related to peptide group INFA-H5-1-V8. Fig. 8 shows an analytical HPLC chromatogram of crude INFA-H5-1-V8 peptide. Fig. 16 shows a MALDI-TOF spectrum of crude INFA-H5-1-V8 peptides.

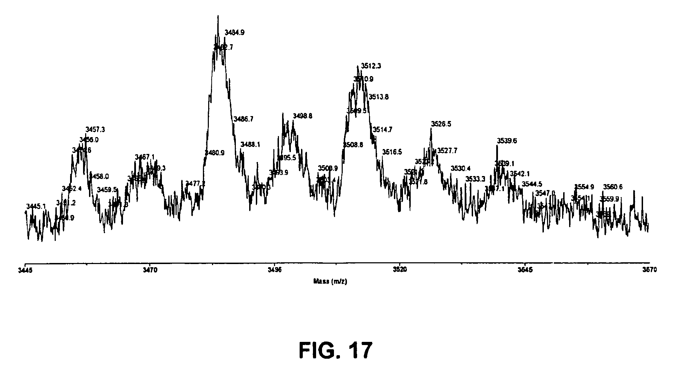

**Fig. 17** shows a MALDI-TOF spectrum of crude lipidated INFA-H5-1-V8 peptide sequences (corresponding to SEQ ID NOs: 213 to 248). SEQ ID NOs 213-248 are lipidated versions of SEQ ID NOs 177-212.

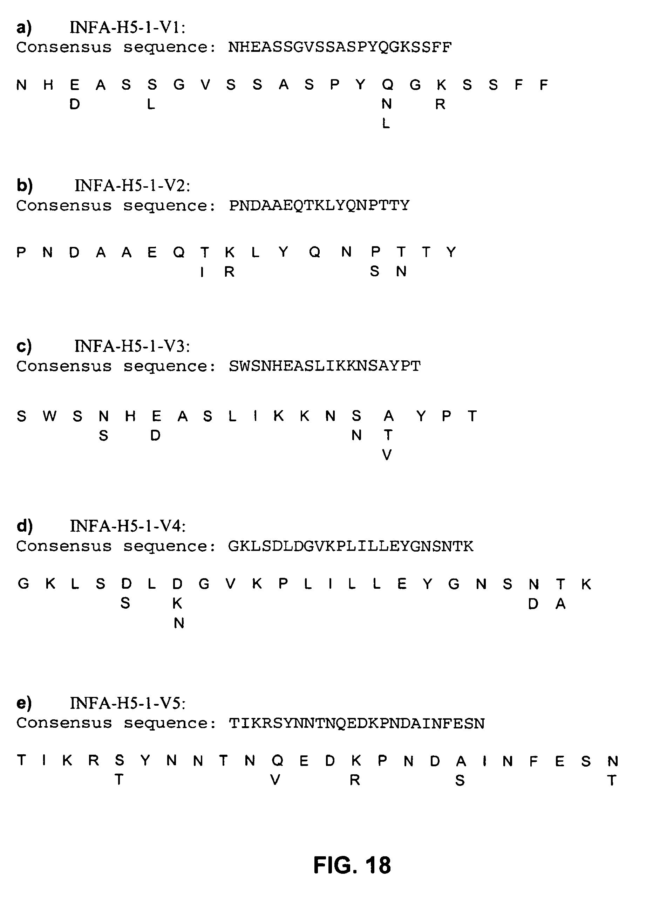

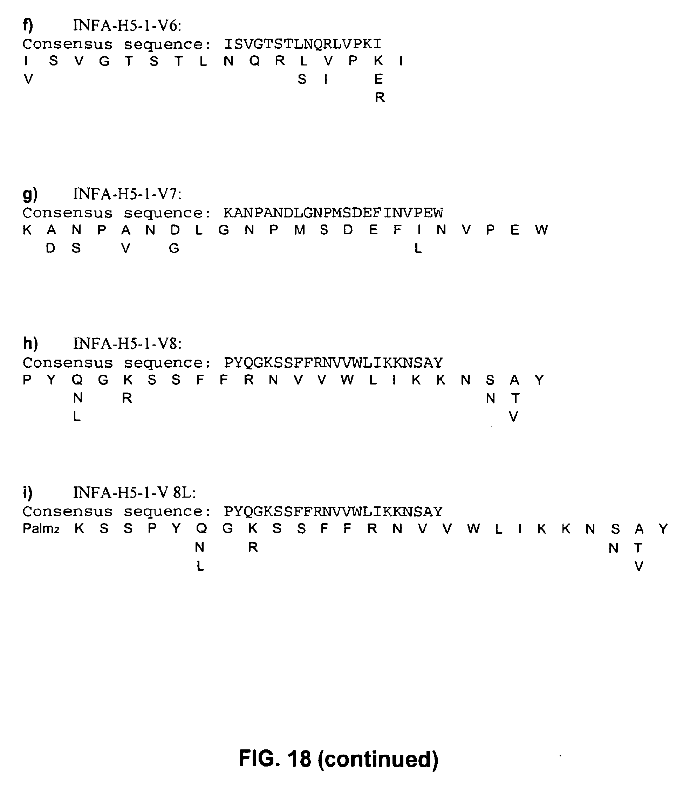

**Fig. 18** shows the variosites of the present invention, including variable amino acid residues. Fig. 18 (a) shows the peptides of group INFA-H5-1-V1. Fig. 18 (b) shows the peptides of INFA-H5-1-V2. Fig. 18 (c) shows the peptides of INFA-H5-1-V3. Fig. 18 (d) shows the peptides of_INFA-H5-1-V4. Fig. 18 (e) shows the peptides of INFA-HS-1-V5. Fig. 18 (f) shows the peptides of INFA-H5-1-V6. Fig. 18 (g) shows the peptides of INFA-H5-1-V7. Fig. 18 (h) shows the peptides of INFA-H5-1-V8.

### Design of vaccine formulations

In the context of the present invention, a vaccine formulation is a cocktail of peptides that are used in the preparation of an influenza vaccine. The vaccine can comprise the cocktail of peptides and other substituents known in the art that would be found acceptable for inclusion. These substituents can include, but are not limited to, adjuvants, diluents and/or carriers.

As used in the present application, a peptide "analogue" can include a variant in which one or more residues are added, deleted, inserted or substituted, while having no material effect on the function of the peptide. That is, a peptide analogue as described should be capable of inducing an antibody or T-cell response to HA. A residue (or residues) may be added or deleted from either end of the peptide, deleted from within the peptide, inserted within the peptide, or substituted for one or more of the residues within the peptide. As would be understood by a person of ordinary skill in art, one or more peptide residues may be added, deleted, inserted or substituted while still maintaining the function of the peptide. For example, as many as five or more residues may be added to or removed from either end of a peptide, or inserted into a peptide, and be considered a peptide analogue within the context of the present invention. In a further example, a conservative substitution of one or more residues within a peptide may result in a peptide analogue. As would be well understood to the skilled artisan, a conservative substitution includes a substitution of one amino acid residue with another amino acid residue having one or more similar chemical properties, such as polarity, charge, hydrophobicity, or aromaticity, for example.

The vaccine formulations of the present invention are particularly suitable for preparing vaccines in the treatment of avian influenza. However, it will be appreciated that any combination of peptide sequences, or formulations comprising these peptide sequences, may be used in other influenza phenotypes.

Peptide vaccines can be prepared with a pool of one or more peptide sequences from SEQ ID NOs: 1 to 212 or SEQ ID NOs: 249 to 460 representing epitopes contained in the three-dimensional structure of HA. The vaccines may further comprise one or more lipidated peptides, including one or more peptides from SEQ ID NOs: 213 to 248 or SEQ ID NOs: 461 to 496. The vaccines may comprise one or more discotope constructs (peptides containing non-variable amino acid residues) or one or more discosite constructs (peptides containing variable amino acid residues). A discosite construct described is derived from one of these epitopes. Thus, a discosite construct formulation comprises one or more peptide sequences derived from the epitope containing the variable residues.

Each discosite construct described represents 2^{x} possible peptide sequences based on x varied residues. For example, a discosite construct having 3 or 4 variable residues represents 2³ = 8 or 2⁴ = 16 sequences, respectively. Therefore, in the context of the present invention, a discosite construct as referred to herein includes the epitope sequence containing the variable residues and the one or more possible sequences derived therefrom.

It will be appreciated by the person of ordinary skill in the art that additional sequences may or may not be added as required.

The vaccine may be prepared by any methodology acceptable to one skilled in the art. For example, oligonucleotides encoding these peptides may be inserted in viral or non-viral vectors for delivery. Peptides may be synthesized individually in and mixed together to accomplish an acceptable formulation. Any variety of different modes by which these peptide antigens may be prepared is acceptable for use with the invention.

In order to formulate a vaccine that is subtype-specific, the proteins selected may contain variable regions with selected variable amino acids that are characteristic of the variability found within the subtype of interest. This allows the vaccine to be subtype specific, which may have the advantage of better representing the antigenic variation among variants within said subtype. To formulate a vaccine that has less subtype distinctiveness, the final peptide formulation may comprise the different subtype specific formulations. For example, a vaccine formulated against avian flu could target variable residues particular to subtype H5 sequences. A vaccine formulated against human flu could target variable residues characteristic of subtypes 1, 2 and/or 3 sequences.

In order to formulate a vaccine that is species-specific, the proteins selected may contain variable regions with selected variable amino acids that are characteristic of the variability found within the species of interest. This allows the vaccine to be species specific, which may have the advantage of better representing the antigenic variation among variants within said species. For example, a vaccine formulated against avian flu could target variable residues particular to avian H5 sequences. A vaccine formulated against human flu could target variable residues characteristic of both, human and avian H5 sequences.

As a specific example, the anti-INF vaccine may include the following isolated peptides: SEQ ID NOs: 1 to 40, SEQ ID NOs: 249 to 288, or peptide analogues thereof, in combination with a pharmaceutically acceptable carrier.

An exemplary anti-INF formulation may comprise one or more of, or all of SEQ ID NOs: 1 to 212, SEQ ID NOs. 249 to 460 or peptide analogues thereof; in combination with a pharmaceutically acceptable carrier. The formulation may also comprise one or more lipidated peptides of SEQ ID NOs: 1 to 212 or SEQ ID NOs: 249 to 460, such as, for example, one or more of SEQ ID NOs: 213 to 248 or SEQ ID NOs: 461 to 496.

Although all peptides of SEQ ID NOs: 1 to 212 or SEQ ID NOs: 249 to 460 may be used in combination as the vaccine formulation, sub-groups of these peptides could be used together.

### EXAMPLES

### Peptide synthesis

The peptides were synthesized by solid phase peptide synthesis (SPPS) using 9-fluoroenylmethoxycarbonyl (Fmoc) chemistry on Pioneer™ automated peptide synthesizer, utilizing pre-loaded Fmoc protected NovaSyn™ TGT resin (NovaBiochem) as described. Where variability at a given position is desired, mixture of two amino acids is placed at that position. This is repeated each time during the synthesis wherever the variability is desired. While 1M solution of 2-(1H-Benzotriazole-1-yl)-1, 1,3,3tetramethyluronium tetrafluoroborate (TBTU) and N-Hydroxybenzotriazole (HOBt) in dimethylformamide (DMF), and 1 M solution of diisopropylethyl amine (DIPEA) in OMF was used for coupling amino acids, 20% piperidine in DMF was used for deblocking amino acids during the synthesis. Coupling was allowed to occur for one hour at room temperature. After the last amino acid was coupled, the resin was taken out from synthesizer and washed on a sintered glass funnel several times with OMF, with 2-propanol and with dichloromethylene (DCM), and dried under high vacuum. The peptide mixtures are cleaved and deprotected by the addition of a solution containing TFA /water/ phenol / thioanisole / EDT /TIS [82:5:5:5:2:1]. The resin was incubated at room temperature for 4 hours. Cleavage mixture was then filtered under reduced pressure into a flask containing a 10-fold volume of cold ether. Resin was also rinsed twice with TFA into the same ether solution. Following incubation for 30 minutes in a freezer to further assist precipitation, the sample was centrifuged at 1 ,000Xg for 5 minutes, and the ether removed. This extraction process was repeated three times. Following a final ether extraction, the residual organic solvent was evaporated under nitrogen gas, and the peptide mixture was redissolved in water and purified by using high performance liquid chromatography (HPLC). Excess of the solvent was removed by using a rotor evaporator, and finally lyophilized to dry powder. Mass spectrometry and amino acid analysis were performed on all the Discotopes to ensure that they have the appropriate peptide content.

Lipidation is performed as follows. Upon completion of the synthesis of a mixed peptide formulation on the synthesiser, the resin is removed from the column and placed into a vial. Dissolve 10 eq. of the Palmitic Acid, 10 eq. of TBTU and 10 eq. HOBT (all relative to the resin) in DMF (10 ml/0.1 mmol resin). Add the solution to the peptidyl resin in the vial. Add 20 eq. (relative to the resin) of the DIPEA. Adjust pH to 8-9 by adding DIPEA drop-wise. Seal the vial with a screw cap and shake the mixture overnight (at least 12 hours).

### Vaccine efficacy

Vaccine formulations comprising the peptide sequences of the present invention were tested in mice. The vaccines used are as follows:
INF-01P consists of two variosites INFA-H5-1-V1 and INFA-H5-1-V2 (SEQ ID NOs: 249-288), also referred as AviFlu-2(Montanide).
INF-02P consists of 8 variosites INFA-H5-1-V1 to INFA-H5-1-V8 (SEQ ID NOs: 249-460), also referred as AviFlu-8(Montanide), AviFlu(Montanide), AviFlu(Alum).
INF-02L consist of 9 variosites INFA-H5-1-V1 to INFA-H5-1-V8L (SEQ ID NOs: 249-496), also referred as AviFlu(Lipidated), AviFlu(Lipidated/Alum).

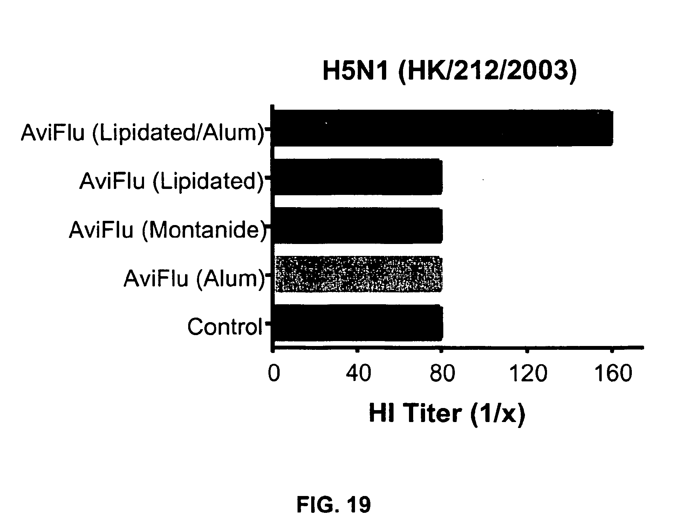

**Fig. 19** illustrates induction of humoral immunity by a vaccine of the present invention after immunization.

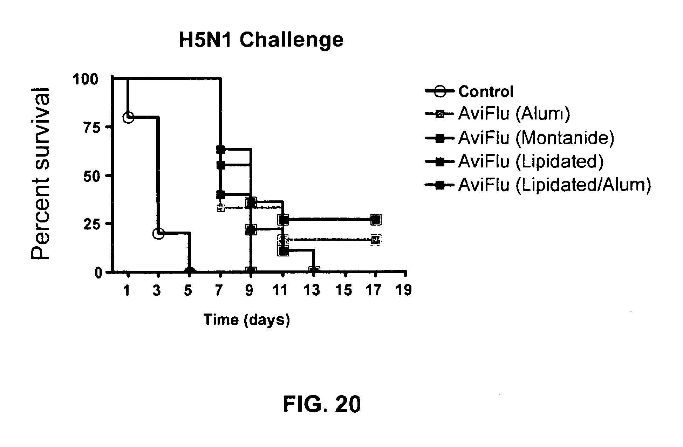

**Fig. 20** illustrates a survival plot of mice vaccinated with a vaccine of the present invention against challenge with H5N1.

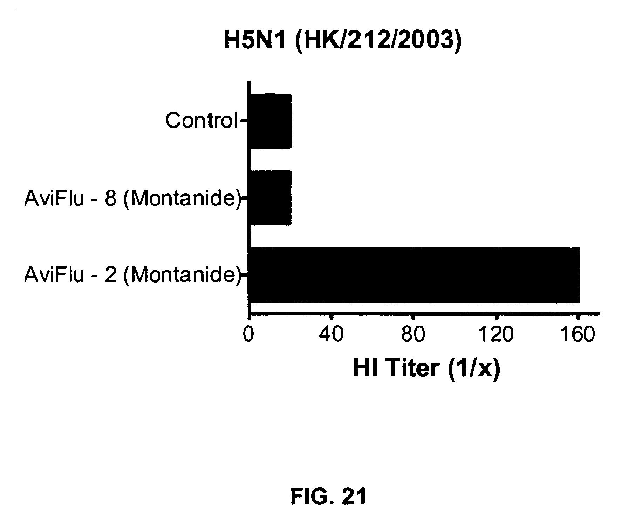

**Fig. 21** shows induction of humoral immunity by INFA-01 P (INFA-HA-1-(V1-V2)) versus INFA-02P (INFA-HA-1-(Vl-V8)) after vaccination in mice as measured by HAI titres.

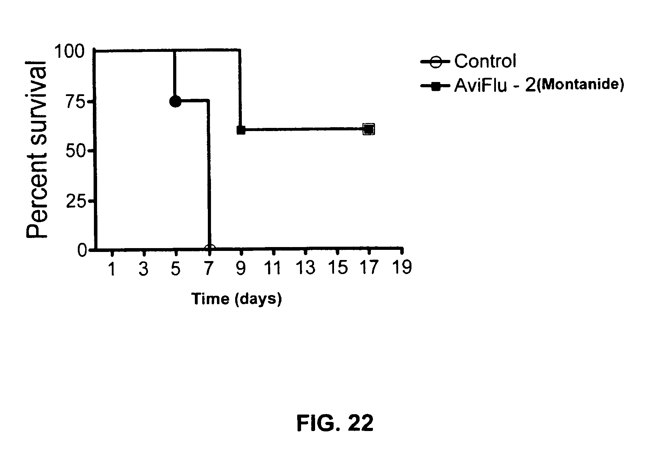

**Fig. 22** shows a survival plot of mice, vaccinated by INFA-01P (INFA-HA-1-(V1-V2)), against challenge with H5N1.

The above-described embodiments of the present invention are intended to be examples only. Alterations, modifications and variations may be effected to the particular embodiments by those of skill in the art without departing from the scope of the invention, which is defined solely by the claims appended hereto.

### References:

1. Philpott, M. et al., Journal of virology (1990), 64(6), 2941-2947.
2. Kaverin, N. et al., Journal of General Virology (2002), 83, 2497-2505.
3. Hioe, C. et al., Journal of Virology (1990), 64(12), 6246-6251.
4. Ha, Y. et al., Proceedings of the National Academy of Sciences, USA (2001), 98, 11181-11186.
5. Macken, C. et al., "The value of a database in surveillance and vaccine selection." in Options for the Control of Influenza IV. A.D.M.E. Osterhaus, N. Cox & A.W. Hampson (Eds.) Amsterdam: Elsevier Science, 2001, 103-106.

## Claims

1. A peptide-based anti-influenza formulation consisting of a mixture of peptides, the sequences of the peptides in the mixture consisting of SEQ ID. NOs: 1 to 40.

2. A vaccine comprising the formulation of claim 1 together with a pharmaceutically-acceptable diluent or carrier.

3. The vaccine of claim 2 further comprising an adjuvant.

4. The vaccine of claim 3 wherein the adjuvant is alum.

5. Use of the formulation of claim 1 for the preparation of a vaccine for preventing or treating influenza in an animal in need thereof.

6. The use according to claim 5 wherein the influenza is avian influenza.

7. The vaccine according to any one of claims 2 to 4 for use in preventing or treating influenza.

8. The vaccine according to claim 7 wherein the influenza is avian influenza.

## Patentansprüche

1. Peptidbasierte Antigrippe-Formulierung, die aus einer Mischung von Peptiden besteht, wobei die Sequenzen der Peptide in der Mischung aus SEQ ID. NO: 1 bis 40 bestehen.

2. Impfstoff, der die Formulierung von Anspruch 1 zusammen mit einem pharmazeutisch akzeptablen Verdünnungsmittel oder einer Trägersubstanz aufweist.

3. Impfstoff gemäß Anspruch 2, der weiterhin einen Hilfsstoff aufweist.

4. Impfstoff gemäß Anspruch 3, wobei der Hilfsstoff Alaun ist.

5. Verwendung der Formulierung von Anspruch 1 zur Herstellung eines Impfstoffes zum Vorbeugen oder Behandeln von Grippe in einem Tier, das dieses benötigt.

6. Verwendung gemäß Anspruch 5, wobei die Grippe Geflügelpest ist.

7. Impfstoff gemäß einem der Ansprüche 2 bis 4 zur Verwendung für die Vorbeugung oder Behandlung von Grippe.

8. Impfstoff gemäß Anspruch 7, wobei die Grippe Geflügelpest ist.

## Revendications

1. Formulation anti-grippe à base de peptides constituée d'un mélange de peptides, les séquences des peptides se trouvant selon le mélange constitué de la séquence à n° d'identification 1 à la séquence à n° d'identification 40.

2. Vaccin comprenant la formulation selon la revendication 1, associé à un diluant ou un vecteur pharmaceutiquement acceptable.

3. Vaccin selon la revendication 2, comprenant en outre un adjuvant.

4. Vaccin selon la revendication 3, dans lequel l'adjuvant est de l'alun.

5. Utilisation de la formulation selon la revendication 1, en vue de la préparation d'un vaccin destiné à prévenir ou à traiter une grippe chez un animal qui en a besoin.

6. Utilisation selon la revendication 5, dans lequel la grippe est une grippe aviaire.

7. Vaccin selon l'une quelconque des revendications 2 à 4, destiné à être utilisé pour empêcher ou traiter une grippe.

8. Vaccin selon la revendication 7, dans lequel la grippe est une grippe aviaire.
